# EUROPEAN PATENT APPLICATION

(11) **EP 2 325 184 A1**
(43) Date of publication of application: **25.05.2011**
(21) Application number: 10181548.8
(22) Date of filing: 29.06.2005
(51) Int. Cl.: C07D 471/04, A61K 31/437, A61P 37/08, A61P 29/00

(54) **Azaindoles useful as inhibitors of protein kinases**

(30) Priority: 30.06.2004 US 584383 P; 01.07.2004 US 584721 P; 04.04.2005 US 98751; 04.04.2005 WO PCT/US2005/011358
(62) Divisional of application: 05769563.7
(71) Applicant: Vertex Pharmceuticals Incorporated, Cambridge, MA 02139-4242 (US)
(72) Inventor: PIERARD, Francoise, Abingdon, Oxfordshire OX 14 4RY (GB); JIMENEZ, Juan-Miguel, Abingdon, Oxfordshire OX14 4RY (GB); KENGTEL, Ronald, Abingdon, Oxfordshire OX14 4RY (GB); BRENCHLEY, Guy, Abingdon, Oxfordshire OX14 4RY (GB); MORTIMORE, Michael, Abingdon, Oxfordshire OX14 4RY (GB); MAZZEI, Francesca, Abingdon, Oxfordshire OX14 4RY (GB)
(74) Representative: Cohausz & Florack

(57) **Abstract**

The present invention relates to pyrrolo [2,3b] pyridine compounds of formula (I) useful as inhibitors of protein kinases. The invention also provides pharmaceutically acceptable compositions comprising said compounds and methods of using the compositions in the treatment of various diseases, conditions, or disorders. The invention also provides processes for preparing the compounds of the invention.

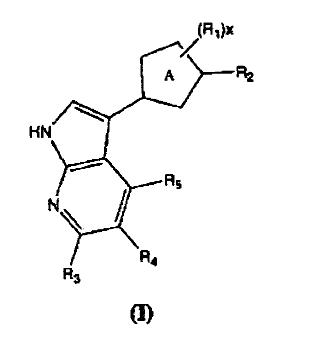

## Description

### TECHNICAL FIELD OF THE INVENTION

The present invention relates to compounds useful as inhibitors of protein kinases. The invention also provides pharmaceutically acceptable compositions comprising the compounds of the invention and methods of using the compositions in the treatment of various disorders. The invention also provides processes for preparing the compounds of the invention.

### BACKGROUND OF THE INVENTION

The search for new therapeutic agents has been greatly aided in recent years by a better understanding of the structure of enzymes and other biomolecules associated with diseases. One important class of enzymes that has been the subject of extensive study is protein kinases.

Protein kinases constitute a large family of structurally related enzymes that are responsible for the control of a variety of signal transduction processes within the cell. (See, Hardie, G. and Hanks, S. The Protein Kinase Facts Book, I and II, Academic Press, San Diego, CA: 1995). Protein kinases are thought to have evolved from a common ancestral gene due to the conservation of their structure and catalytic function. Almost all kinases contain a similar 250-300 amino acid catalytic domain. The kinases may be catogorized into families by the substrates they phosphorylate (e.g., protein-tyrosine, protein-serine/threonine, lipids, etc.). Sequence motifs have been identified that generally correspond to each of these kinase families (See, for example, Hanks, S.K., Hunter, T., FASEB J. 1995, 9, 576-596; Knighton et al.. Science 1991, 253, 407-414; Hiles et al., Cell 1992, 70, 419-429; Kunz. et al., Cell 1993, 73, 585-596; Garcia-Bustos et al., EMBO J. 1994, 13, 2352-2361).

[0103] In general, protein kinases mediate intracellular signaling by effecting a phosphoryl transfer from a nucleoside triphosphate to a protein acceptor that is involved in a signaling pathway. These phosphorylation events act as molecular on/off switches that can modulate or regulate the target protein biological function. These phosphorylation events are ultimately triggered in response to a variety of extracellular and other stimuli. Examples of such stimuli include environmental and chemical stress signals (e.g., osmotic shock, heat shock, ultraviolet radiation, bacterial endotoxin, and HiO₂), cytokines (e.g., interleukin (IL-1) and tumor necrosis factor α (TNF-α)), and growth factors (e.g., granulocyte macrophage-colony-stimulating factor (GM-CSF), and fibroblast growth factor (FGF)). An extracellular stimulus may affect one or more cellular responses related to cell growth, migration, differentiation, secretion of hormones, activation of transcription factors, muscle contraction, glucose metabolism, control of protein synthesis, and regulation of the cell cycle.

Many diseases are associated with abnormal cellular responses triggered by protein kinase-mediated events as described above. These diseases include, but are not limited to, autoimmune diseases, inflammatory diseases, bone diseases, metabolic diseases, neurological and neurodegenerative diseases, cancer, cardiovascular diseases, allergies and asthma, Alzheimer's disease, and hormone-related diseases. Accordingly, there has been a substantial effort in medicinal chemistry to find protein kinase inhibitors that are effective as therapeutic agents.

The Tec family of non-receptor tyrosine kinases plays a central role in signaling through antigen-receptors such as the TCR, BCR and Feε receptors (reviewed in Miller A, et al. Current Opinion in Immunology 14;331-340 (2002). Tec family kinases are essential for T cell activation. Three members of the Tec family, Itk, Rlk and Tec, are activated downstream of antigen receptor engagement in T cells and transmit signals to downstream effectors, including PLC-γ. Deletion of Itk in mice results in reduced T cell receptor (TCR)-induced proliferation and secretion of the cytokines IL-2, IL-4, IL-5, IL-10 and IFN-γ (Schaeffer et al, Science 284; 638-641 (1999)), Fowell et al, Immunity 11;399-409 (1999), Schaeffer et al Nature Immunology 2,12; 1183-1188 (2001))). The immunological symptoms of allergic asthma are attenuated in Itk-/- mice. Lung inflammation, eosinophil infiltration and mucous production are drastically reduced in Itk-/- mice in response to challenge with the allergen OVA (Mueller et al, Journal of Immunology 170: 5056-5063 (2003)). Itk has also been implicated in atopic dermatitis. This gene has been reported to be more highly expressed in peripheral blood T cells from patients with moderate and/or severe atopic dermatitis than in controls or patients with mild atopic dermatitis (Matsumoto et al, International archives of Allergy and Immunology 129; 327-340 (2002)).

Splenocytes from Rlk-/- mice secrete half the IL-2 produced by wild type animals in response to TCR engagement (Schaeffer et al, Science 284; 638-641 (1999)), while combined deletion of Itk and Rlk in mice leads to a profound inhibition of TCR-induced responses including proliferation and production of the cytokines IL-2, IL-4, IL-5 and IFN-γ (Schaeffer et al Nature Immunology 2,12; 1183-1188 (2001)), Schaeffer et al, Science 284; 638-641 (1999)). Intracellular signaling following TCR engagement is effected in Itk/Rlk deficient T cells; inositol triphosphate production, calcium mobilization, MAP kinase activation, and activation of the transcription factors NFAT and AP-1 are all reduced (Schaeffer et al, Science 284; 638-641 (1999), Schaeffer et al Nature Immunology 2,12; 1183-1188 (2001)).

Tec family kinases are also essential for B cell development and activation. Patients with mutations in Btk have a profound block in B cell development, resulting in the almost complete absence of B lymphocytes and plasma cells, severely reduced Ig levels and a profound inhibition of humoral response to recall antigens (reviewed in Vihinen et al Frontiers in Bioscience 5:d917-928). Mice deficient in Btk also have a reduced number of peripheral B cells and greatly decreased levels of IgM and IgG3. Btk deletion in mice has a profound effect on B cell proliferation induced by anti-IgM, and inhibits immune responses to thymus-independent type II antigens (Ellmeier et al, J Exp Med 192:1611-1623 (2000)).

Tec kinases also play a role in mast cell activation through the high-affinity IgE receptor (FcεRI). Itk and Btk are expressed in mast cells and are activated by FceRl cross-linking (Kawakami et al, Journal of Immunology; 3556-3562 (1995)). Btk deficient murine mast cells have reduced degranulation and decreased production of proinflammatory cytokines following FcεRI cross-linking (Kawakami et al. Journal of leukocyte biology 65:286-290). Btk deficiency also results in a decrease of macrophage effector functions (Mukhopadhyay et al, Journal of Immunology; 168, 2914-2921 (2002)).

The Janus kinases (JAK) are a family of tyrosine kinases consisting of JAK1, JAK2, JAK3 and TYK2. The JAKs play a critical role in cytokine signaling. The downstream substrates of the JAK family of kinases include the signal transducer and activator of transcription (STAT) proteins. JAK/STAT signaling has been implicated in the mediation of many abnormal immune responses such as allergies, asthma, autoimmune diseases such as transplant rejection, rheumatoid arthritis, amyotrophic lateral sclerosis and multiple sclerosis as well as in solid and hematologic malignancies such as leukemias and lymphomas. The pharmaceutical intervention in the JAK/STAT pathway has been reviewed [Frank Mol. Med. 5, 432-456 (1999) & Scidel, et al, Oncogene 19, 2645-2656 (2000)].

JAK1, JAK2, and TYK2 are ubiquitously expressed, while JAK3 is predominantly expressed in hematopoietic cells. JAK3 binds exclusively to the common cytokine receptor gamma chain (γ_{c}) and is activated by IL-2, IL-4, IL-7, IL-9, and IL-15. The proliferation and survival of murine mast cells induced by IL-4 and IL-9 have, in fact, been shown to be dependent on JAK3- and γ_{c}- signaling [Suzuki et al, Blood 96, 2172-2180 (2000)].

Cross-linking of the high-affinity immunoglobulin (1g) E receptors of sensitized mast cells leads to a release of proinflammatory mediators, including a number of vasoactive cytokines resulting in acute allergic, or immediate (type I) hypersensitivity reactions [Gordon et al, Nature 346, 274-276 (1996) & Galli, N. Engl. J. Med., 328, 257-265 (1993)]. A crucial role for JAK3 in IgE receptor-mediated mast cell responses *in vitro* and *in vivo* has been established [Malaviya, et al, Biochem. Biophys. Res. Commun. 257, 807-813 (1999)]. In addition, the prevention of type I hypersensitivity reactions, including anaphylaxis, mediated by mast cell-activation through inhibition of JAK3 has also been reported [Malaviya et al, J. Biol. Chem. 274,27028-27038 (1999)]. Targeting mast cells with JAK3 inhibitors modulated mast cell degranulation *in vitro* and prevented IgE receptor/antigen-mediated anaphylactic reactions *in vivo.*

A recent study described the successful targeting of JAK3 for immune suppression and allograft acceptance. The study demonstrated a dose-dependent survival of Buffalo heart allograft in Wistar Furth recipients upon administration of inhibitors of JAK3 indicating the possibility of regulating unwanted immune responses in graft versus host disease [Kirken, Transpl. Proc. 33, 3268-3270 (2001)].

IL-4-mediated STAT-phosphorylation has been implicated as the mechanism involved in early and late stages of rheumatoid arthritis (RA). Up-regulation of proinflammatory cytokines in RA synovium and synovial fluid is a characteristic of the disease. It has been demonstrated that IL-4-mediated activation of IL-4/STAT pathway is mediated through the Janus Kinases (JAK 1 & 3) and that IL-4-associated JAK kinases are expressed in the RA synovium [Muller-Ladner, et al, J. Immunol. 164, 3894-3901 (2000)].

Familial amyotrophic lateral sclerosis (FALS) is a fatal neurodegenerative disorder affecting about 10% of ALS patients. The survival rates of FALS mice were increased upon treatment with a JAK3 specific inhibitor. This suggested that JAK3 plays a role in FALS [Trieu, et al, Biochem. Biophys. Res. Commun. 267, 22-25 (2000)].

Signal transducer and activator of transcription (STAT) proteins are activated by, among others, the JAK family kinases. Results form a recent study suggested the possibility of intervention in the JAK/STAT signaling pathway by targeting JAK family kinases with specific inhibitors for the treatment of leukemia [Sudbeck, et al, Clin. Cancer Res. 5, 1569-1582 (1999)]. JAK3 specific compounds were shown to inhibit the clonogenic growth of JAK3-expressing cell lines DAUDI, RAMOS, LC1;19, NALM-6, MOLT-3 and HL-60.

In animal models, TEL/JAK2 fusion proteins have induced myeloproliferative disorders and in hematopoietic cell lines, introduction of TEL/JAK2 resulted in activation of STAT1, STAT3, STAT5, and cytokine-independent growth [Schwaller, et al, EMBO J. 17, 5321-5333 (1998)].

Inhibition of JAK 3 and TYK 2 abrogated tyrosine phosphorylation of STAT3, and inhibited cell growth of mycosis fungoides, a form of cutaneous T cell lymphoma. These results implicated JAK family kinases in the constitutively activated JAK/STAT pathway that is present in mycosis fungoides [Nielsen, et al, Proc. Nat. Acad. Sci. U.S.A. 94, 6764-6769 (1997)]. Similarly, STAT3, STAT5, JAK1 and JAK2 were demonstrated to be constitutively activated in mouse T cell lymphoma characterized initially by LCK over-expression, thus further implicating the JAK/STAT pathway in abnormal cell growth [Yu, et al, J. Immunol. 159, 5206-5210 (1997)]. In addition, IL-6 mediated STAT3 activation was blocked by an inhibitor of JAK, leading to sensitization of myeloma cells to apoptosis [Catlett-Falcone, et al, Immunity 10, 105-115 (1999)].

Accordingly, there is a great need to develop compounds useful as inhibitors of protein kinases. In particular, it would be desirable to develop compounds that are useful as inhibitors of Tec family (e.g.,Tec, Btk, Itk/Emt/Tsk, Bmx, Txk/Rlk) and JAK family protein kinases, particularly given the inadequate treatments currently available for the majority of the disorders implicated in their activation.

### SUMMARY OF THE INVENTION

It has now been found that compounds of this invention, and pharmaceutically acceptable compositions thereof, are effective as inhibitors of protein kinases. In certain embodiments, these compounds are effective as inhibitors Tec family (e.g.,Toc, Btk, Itk/Emt/Tsk, Bmx, Txk/RIk) protein kinases and/or JAK kinases. These compounds have the general formula I as defined herein or a pharmaceutically acceptable salt thereof.

These compounds and pharmaceutically acceptable compositions thereof are useful for treating or preventing a variety of diseases, disorders or conditions, including, but not limited to, an autoimmune, inflammatory, proliferative, or hyperproliferative disease or an immunologically-mediated disease. The compositions are also useful in methods for preventing thrombin-induced platelet aggregation. The compounds provided by this invention are also useful for the study of kinases in biological and pathological phenomena; the study of intracellular signal transduction pathways mediated by such kinases; and the comparative evaluation of new kinase inhibitors.

### DETAILED DESCRIPTION OF THE INVENTION

### 1. General Description of Compounds of the Invention -

The present invention relates to a compound of formula **I:** 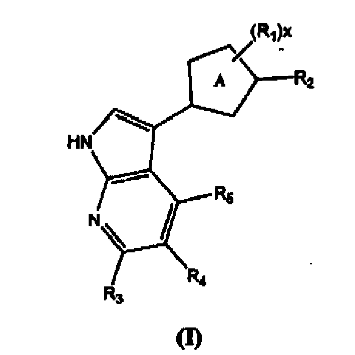 or a pharmaceutically acceptable salt thereof, wherein:
Ring A is an optionally substituted five membered ring selected from: 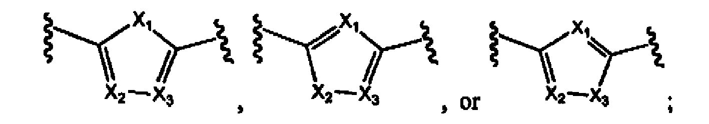 x is 0, 1 or 2;
   each occurrence of R¹ is independently halogen, CN, NO₂, or UₘR;
   R² is independently selected from Tₙ-R'
   X¹, X² and X³ are each independently CR¹, N, S or O;
   R³, R⁴, and R⁵ are each independently halogen, CN, NO₂, or Vₚ-R';
   each occurrence of T, U or V is independently an optionally substituted C₁₋₆ alkylidene chain, wherein up to two methylene units of the chain are optionally and independently replaced by NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-;
m , n and p are each independently 0 or 1;
   each occurrence of R is independently hydrogen or an optionally substituted C₁₋₆ aliphatic group; and each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R', two occurrences of R, or two occurrences of R', are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur; provided that at least one occurrence of R³, R⁴, R⁵ is Vₚ-R', wherein R' is not hydrogen.
For the avoidance of doubt, (R¹)ₖ, if present, is bound at any position of Ring A except for the position occupied by R²

In one embodiment,
a) if n is 0, then R' is not H;
b) if Ring A is 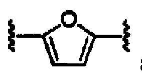 and R⁴ is 2-phenoxylphenyl, then R² is not COOH or CONHR^{x} wherein R^{x} is *n*-propyl, phenyl, cyclohexyl, benzyl, -CH₂CH₂OH, -CHₓ-cycloproyl, -CH₂CH₂OCH₃, 3-pyridyl, 4-hydroxy-cyclohexyl, or -CH₂-C=CH.

In another embodiment, the compounds of this invention do not include the compounds listed in claim 9 on pages 152-166 of WO2004/078756 A2, which is hereby incorporated by reference.

### 2. Compounds and Definitions:

Compounds of this invention include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

As described herein, compounds of the invention may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted" In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

As described herein, a specified number of atoms includes any integer therein. For example, a group having from 1-4 atoms, could have 1,2,3, or 4 atoms.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic C₃-C₈ hydrocarbon or bicyclic C₈-C₁₂ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "heteroaliphatic", as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle", "heterocyclyl", "heterocycloaliphatic", or "heterocyclic" groups.

The term "heterocycle", "heterocyclyl", "heterocycloaltphatic", or "heterocyclic" as used herein means non-aromatic, monocyclic, bicyclic, or tricyclic ring systems in which one or more ring members are an independently selected heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members. Suitable heterocycles include, but are not limited to, 3-1H-benzimidazol-2-one, 3-(1-alkyl)-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-tetrahydropiperazinyl, 2-tetahydrooperazinyl, 3-tetrahydropiperazinyl, 1-piperidinyl, 2-piperidinyl, 3-piperidinyl, 1-pyrazolinyl, 3-pyrazolinyl, 4-pyrazolinyl, 5-pyrazolinyl, 1-piperidinyl, 2-piperidinyl, 3-piperidinyl, 4-piperidinyl, 2-thiazolidinyl, 3-thiazolidinyl, 4-thiazolidinyl, 1-imidazolidinyl, 2-imidazolidinyl, 4-imidazolidinyl, 5-imidazolidinyl, indolinyl, tetrahydroqumolinyl, tetrahydroisoquinolinyl, benzothiolane, benzodithiane, and 1,3-dihydro-imidazol-2-one.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or, a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term "unsaturated", as used herein, means that a moiety has one or more units of unsaturation.

The term "alkoxy", or "thioalkyl", as used herein, refers to an alkyl group, as previously defined, attached to the principal carbon chain through an oxygen ("alkoxy") or sulfur ("thioalkyl") atom.

The terms "haloalkyl", "haloalkenyl" and "haloalkoxy" means alkyl, alkenyl or alkoxy, as the case may be, substituted with one or more halogen atoms. The term "halogen" means F, Cl, Br, or I.

The term "acryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring".

The term "heteroaryl", used alone or as part of a larger moiety as in "heteroaralkyl" or "heteroarylalkoxy", refers to monocyclic, bicyclic, and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic". Suitable heteroaryl rings include, but are not limited to, 2-furanyl, 3-furanyl, N-imidazolyl, 2-imidazolyl, 4-imidazolyl, 5-imidazolyl, benzimidazolyl, 3-isoxazolyl, 4-isoxazolyl, 5-isoxazolyl, 2-oxazolyl, 4-oxazolyl, 5-oxazolyl, N-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thianyl, benzofuryl, benzothiophenyl, indolyl (e.g., 2-indolyl), pyrazolyl (e.g., 2-pyrazolyl), isothiazolyl, 1,2,3-oxadiazolyl, 1,2,5-oxadiazolyl, 1,2,4-oxadiazolyl, 1,2,3-triaxolyl, 1,2,3-thiadiazolyl, 1,3,4-thiadiazolyl, 1,2,5-thiadiazolyl, purinyl, pyrazinyl, 1,3,5-triazinyl, quinolinyl (e.g., 2-quinolinyl, 3-quinolinyl, 4-quinolinyl), and isoquinolinyl (e.g., 1-isoquinolinyl, 3-isoquinolinyl, or 4-isoquinolinyl).

An aryl (including aralkyl, aralkoxy, aryloxyalkyl and the like) or heteroaryl (including heteroaralkyl and heteroarylalkoxy and the like) group may contain one or more substituents. Suitable substituents on the unsaturated carbon atom of an aryl or heteroaryl group are selected from halogen; -R°; -OR°; -SR°; 1,2-methylenedioxy-, 1,2-ethylenedioxy; phenyl (Ph) optionally substituted with R°; -O(Ph) optionally substituted with R°; -(CH₂)₁₋₂(Ph), optionally substituted with R°; -CH=CH(Ph), optionally substituted with R°; -NO₂; -CN; -N(R°)₂; NR°C(O)R°; NR°C(S)R°; -NR°C(O)N(R°)₂; -NR°C(S)N(R°)₂; -NR°CO₂R°; -NR°NR°C(O)R°; -NR°NR°C(O)N(R°)₂; -NR°NR°CO₂R°; -C(O)C(O)R°; -C(O)CH₂C(O)R°; -CO₂R°; -C(O)R°; -C(S)R°; -C(O)N(R°)₂; -C(S)N(R°)₂; -OC(O)N(R°)₂; -OC(O)R°; -C(O)N(OR°)R°; -C(NOR°)R°; -S(O)₂R°; -S(O)₃R°; -SO₂N(R°)₂; -S(O)R°; -NR°SO₂N(R°)₂; NR°SO₂R°; -N(OR°)R°; -C(=NH)-N(R°)₂; or -(CH₂)₀₋₂NHC(O)R° wherein each independent occurrence of R° is selected from hydrogen, optionally substituted C₁₋₆ aliphatic, an unsubstituted 5-6 membered heteroaryl or heterocyclic ring, phenyl, -O(Ph), or -CH₂(Ph), or, notwithstanding the definition above, two independent occurrences of R°, on the same substituent or different substituents, taken together with the atom(s) to which each R° group is bound, form a 5-8-membered heterocyclyl, aryl, or heteroaryl ring or a 3-8-membered cycloalkyl ring having 0-3 heteroatoms independently selected from nitrogen, oxygen, or sulfur. Optional substituents on the aliphatic group of R° are selected from NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic, wherein each of the foregoing C₁₋₄aliphatic groups of R° is unsubstituted.

An aliphatic or hateroaliphatic group, or a non-aromatic heterocyclic ring may contain one or more substituents. Suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR*, =NN(R*)₂, =NNHC(O)R*, -NNHCO₂(alkyl), =NNHSO₂(alkyl), or -NR*, where each R* is independently selected from hydrogen or an optionally substituted C₁₋₆ aliphatic. Optional substituents on the aliphatic group of R* are selected from NH₂, NH(C₁₋₄ aliphatic), N(C₁₋₄ aliphatic)₂, halogen, C₁₋₄ aliphatic, OH, O(C₁₋₄ aliphatic), NO₂, CN, CO₂H, CO₂(C₁₋₄ aliphatic), O(halo C₁₋₄ aliphatic), or halo(C₁₋₄ aliphatic), wherein each of the foregoing C₁₋₄aliphatic groups of R* is unsubstituted.

Optional substituents on the nitrogen of a non-aromatic heterocyclic ring are selected from R⁺, -N(R⁺)₂, -C(O)R⁺, -CO₂R⁺, -C(O)C(O)R⁺, -C(O)CH₂C(O)R⁺, -SO₂R⁺, -SO₂N(R⁺)₂, -C(=S)N(R⁺)₂, -C(=NH)-N(R⁺)₂, or -NRSO₂R⁺; wherein R⁺ is hydrogen, an optionally substituted C₁₋₆ aliphatic, optionally substituted phenyl, optionally substituted -O(Ph), optionally substituted -CH₂(Ph), optionally substituted -(CH₂)₁₋₂(Ph); optionally substituted -CH-CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of R⁺, on the same substituent or different substituents, taken together with the atom(s) to which each R⁺ group is bound, form a 5-8-membered heterocyclyl, aryl, or heteroaryl ring or a 3-8-membered cycloalkyl ring having 0-3 heteroatoms independently selected from nitrogen, oxygen, or sulfur. Optional substituents on the aliphatic group or the phenyl ring of R⁺ are selected from NH₂, NH(C₁₋₄ aliphatic), N(C₁₋₄ aliphatic)₂, halogen, C₁₋₄ aliphatic, OH, O(C₁₋₄ aliphatic), NO₂, CN, CO₂H, CO₂(C₁₋₄ aliphatic), O(halo C₁₋₄ aliphatic), or halo(C₁₋₄ aliphatic), wherein each of the foregoing C₁₋₄aliphatic groups of R⁺ is unsubstituted.

The term "alkylidene chain" refers to a straight or branched carbon chain that may be fully saturated or have one or more units of unsaturation and has two points of attachment to the rest of the molecule, wherein one or more methylene units may optionally and independently be replaced with a group including, but not limited to, CO, CO₂, COCO, CONR, OCONR, NRNR, NRNRCO, NRCO, NRCO₂, NRCONR, SO, SO₂, NRSO₂, SO₂NR, NRSO₂NR, O, S; or NR.

The term "protecting group", as used herein, refers to an agent used to temporarily block one or more desired reactive sites in a multifunctional compound. In certain embodiments, a protecting group has one or more, or preferably all, of the following characteristics a) reacts selectively in good yield to give a protected substrate that is stable to the reactions occurring at one or more of the other reactive sites; and b) is selectively removable in good yield by reagents that do not attack the regenerated functional group. Exemplary protecting groups are detailed in Greene, T.W., Wuts, P. G in "Protective Groups in Organic Synthesis", Third Edition, John Wiley & Sons, New York: 1999, the entire contents of which are hereby incorporated by reference. The term "nitrogen protecting group", as used herein, refers to an agents used to temporarily block one or more desired nitrogen reactive sites in a multifunctional compound. Preferred nitrogen protecting groups also possess the characteristics exemplified above, and certain exemplary nitrogen protecting groups are also detailed in Chapter 7 in Greene, T.W., Wuts, P. G in "Protective Groups in Organic Synthesis", Third Edition, John Wiley & Sons, New York: 1999, the entire contents of which are hereby incorporated by reference.

As detailed above, in some embodiments, two independent occurrences of R° (or R⁺, or any other variable similarly defined herein), are taken together with the atom(s) to which each variable is bound to form a 5-8-membered heterocyclyl, aryl, or heteroaryl ring or a 3-8-membered cycloalkyl ring having 0-3 heteroatoms independently selected from nitrogen, oxygen, or sulfur. Exemplary, rings that are formed when two independent occurrences of R° (or R⁺, or any other variable similarly defined herein) are taken together with the atom(s) to which each variable is bound include, but are not limited to the following: a) two independent occurrences of R° (or R⁺, or any other variable similarly defined herein) that are bound to the same atom and are taken together with that atom to form a ring, for example, N(R°)₂, where both occurrences of R° are taken together with the nitrogen atom to form a piperidin-1-yl, piperazin-1-yl, or morpholin-4-yl group; and b) two independent occurrences of R° (or R⁺, or any other variable similarly defined herein) that are bound to different atoms and are taken together with both of those atoms to form a ring, for example where a phenyl group is substituted with two occurrences of OR° 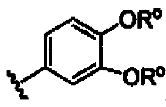 , these two occurrences of R° are taken together with the oxygen atoms to which they are bound to form a fused 6-membered oxygen containing ring 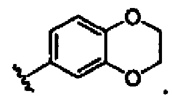 It will be appreciated that a variety of other rings can be formed when two independent occurrences of R° (or R⁺, or any other variable similarly defined herein) are taken together with the atom(s) to which each variable is bound and that the examples detailed above are not intended to be limiting.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enrichad carbon are within the scope of this invention. Such compounds are useful, for example, as analytical tools or probes in biological assays.

### 3. Description of Exemplary Compounds:

All descriptions of embodiments herein may apply to compounds of formula **I**, **II, III, IV, V, and VI.**

In certain embodiments of this invention, R⁴ and R⁵ are each independently Vₚ-R'.

In other embodiments, one of R³, R⁴, and R⁵ is Vₚ-R', wherein, R' is an optionally substituted 5- or 6-membered fully unsaturated (i.e., aromatic) monocyclic ring having 0-3 heteroatoms independently selected from nitrogen, oxygen, or sulfur, or an optionally substituted 9- or 10-membered fully unsaturated bicyclic ring system having 0-5 heteroatoms independently selected from nitrogen, oxygen, or sulfur.

In other embodiments, one of R³, R⁴, and R⁵ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur.

In other embodiments, R⁴ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur. In certain of these embodiments, R³ and R⁵ is Vₚ-R', wherein p is 0 and R' is hydrogen.

In other embodiments, R⁴ is Vₚ-R', wherein R' is independently an optionally substituted C₁₋₆ aliphatic group or an optionally substituted 3-8-membered saturated, partially unsaturated, or fully unsaturated monocyclic ring having 0-3 heteroatoms independently selected from nitrogen, oxygen, or sulfur.

In other embodiments, R⁴ is Vₚ-R', and R' is an optionally substituted a 3-8-membered saturated, partially unsaturated, or fully unsaturated monocyclic ring having 0-3 heteroatoms independently selected from nitrogen, oxygen, or sulfur.

In other embodiments, R⁴ is Vₚ-R', wherein R' is independently an optionally substituted a 5-6-membered fully unsaturated monocyclic ring having 0-3 heteroatoms independently selected from nitrogen, oxygen, or sulfur.

In other embodiments, R⁴ is Vₚ-R', and R' is an optionally substituted a 6-membered fully unsaturated monocyclic ring having 0-3 nitrogen heteroatoms or having 0-1 nitrogen heteroatoms.

In other embodiments, R⁴ is Vₚ-R', and R' is an optionally substituted a 3-8-membered saturated monocyclic ring having 0-3 heteroatoms independently selected from nitrogen, oxygen, or sulfur.

In other embodiments, R⁴ is Vₚ-R', and R' is an optionally substituted a 6-membered saturated monocyclic ring having 0-2 heteroatoms independently selected from nitrogen, oxygen, or sulfur.

In yet other embodiments, R⁴ is Vₚ-R', and R' is an optionally substituted C₁₋₆ aliphatic group. In certain embodiments, R' is C₁₋₆alkynyl. In certain embodiments, R' is -C≡CH.

In other embodiments, p is 0.

In yet other embodiments, p is 1.

In other embodiments, V is NR-, -S-, or -O-.

In other embodiments, R³ is Vₚ-R', wherein p is 0 and R' is hydrogen.

In other embodiments, R⁵ is halogen or Vₚ-R', wherein p is 0 and R' is hydrogen or C₁₋₆ aliphatic. In yet other embodiments, this C₁₋₆ aliphatic is C₁₋₃ alkyl.

In other embodiments, Ring A is: 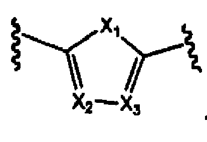 . In certain of these embodiments, X₂ is CR¹.

In other embodiments, Ring A is:

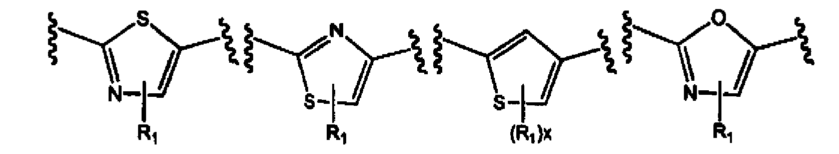

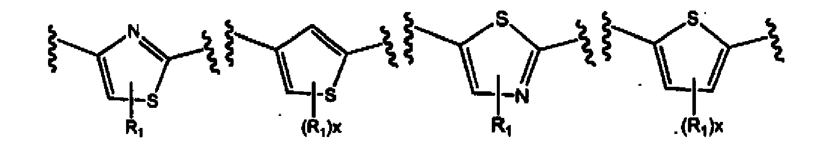

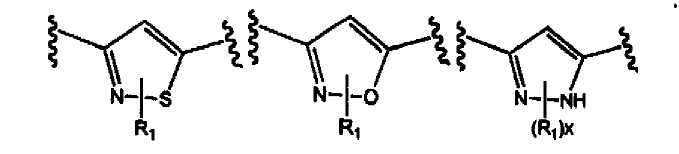

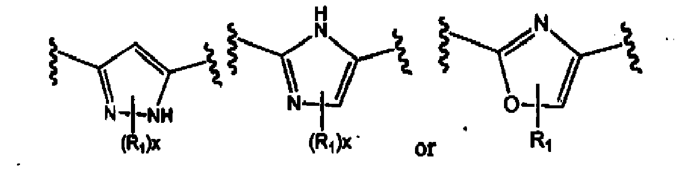

In other embodiments, Ring A is:

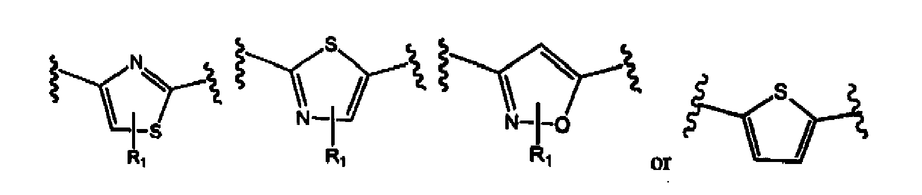

In certain embodiments, R¹ is UₘR. In other embodiments, R¹ is UₘR, wherein m is 0 and R is H or CH₃.

In certain embodiments, R² is TₙR', wherein n is 1.

In other embodiments, R² is TₙR', wherein n is 0.

In certain embodiments, T is NR-, -O-, -CO-, -CONR-, or -NRCO-.

In certain embodiments, T is NR-. In certain embodiments, T is -Q-. In certain of these embodiments, R' is C₁₋₆aliphatic. In other of these embodiments, both R and R' are H.

In certain embodiments, T is -NR- and R' is C₁₋₆aliphatic. In certain of these embodiments, R is C₁₋₆aliphatic. In some embodiments, both R and R' are C₁₋₆alkyl.

In certain embodiments, T is a C₁₋₆ alkylidene chain wherein the alkylidene chain is attached to Ring A through a methylene unit. In some of these embodiments, T is -(C₁₋₅alkyl)NR- In some embodiments, T is-CH₂NR-. In some of these embodiments, R' is C₁₋₆aliphatic.

In other embodiments, T is a C₁₋₆ alkylidene chain wherein 0 methylene units are replaced with the groups disclosed herein.

In yet other embodiments, R² is an optionally substituted 5-7 membered N-attached heterocyclyl. In certain embodiments, said N-attached heterocyclyls are selected from morpholinyl, piperidinyl, pyrrolidinyl, and piperazinyl. In certain embodiments, said N-attached heterocyclyls are optionally and independently substituted with 0-4 occurrences of amino, alkyl amino, dialkylamino, or C₁₋₆alkyl.

As described generally above, another compound of this invention has the formula II: 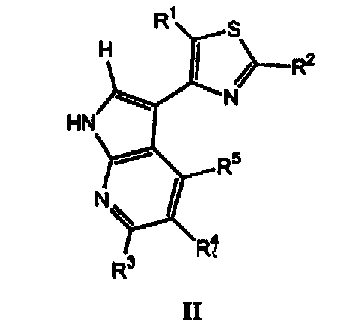 or a pharmaceutically acceptable salt thereof.

In other embodiments, a compound of this invention has the formula **III**: 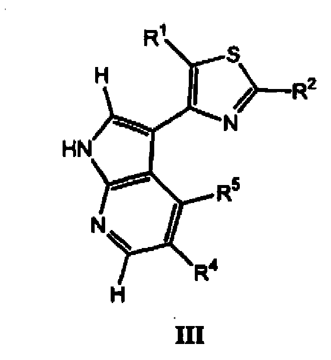 or a pharmaceutically acceptable salt thereof.

In still other embodiments, a compound has the formula **IV:** 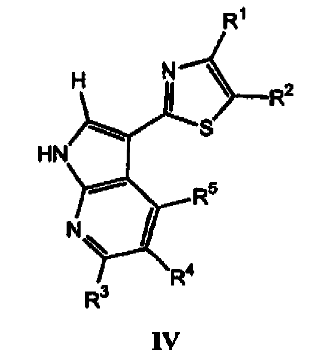 or a pharmaceutically acceptable salt thereof.

In still other embodiments, a compound has the formula **V**: 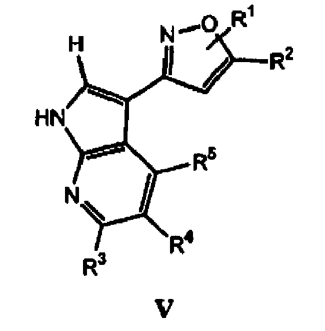 or a pharmaceutically acceptable salt thereof.

In still other embodiments, a compound has the formula **VI:** 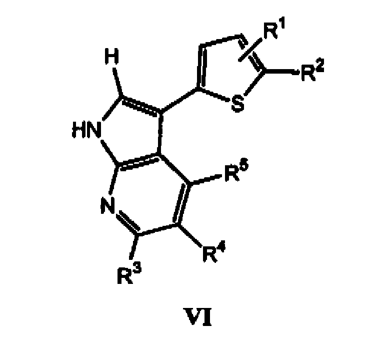 or a pharmaceutically acceptable salt thereof.

It will be appreciated that for compounds of formulae **II-VI** the variables in the formulae **II-VI** compounds are as defined in any of the embodiments herein.

As described generally above, preferred substituents and variables (e.g., R' groups) are as exemplified in the compounds depicted in Table 1.

Accordingly, representative examples of compounds of formula **I** are depicted below in Tables 1 and 2.

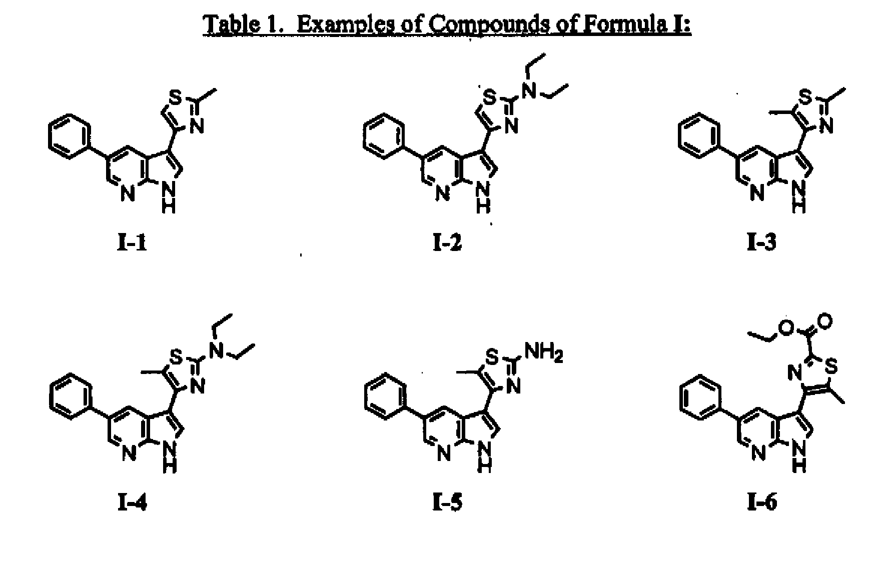

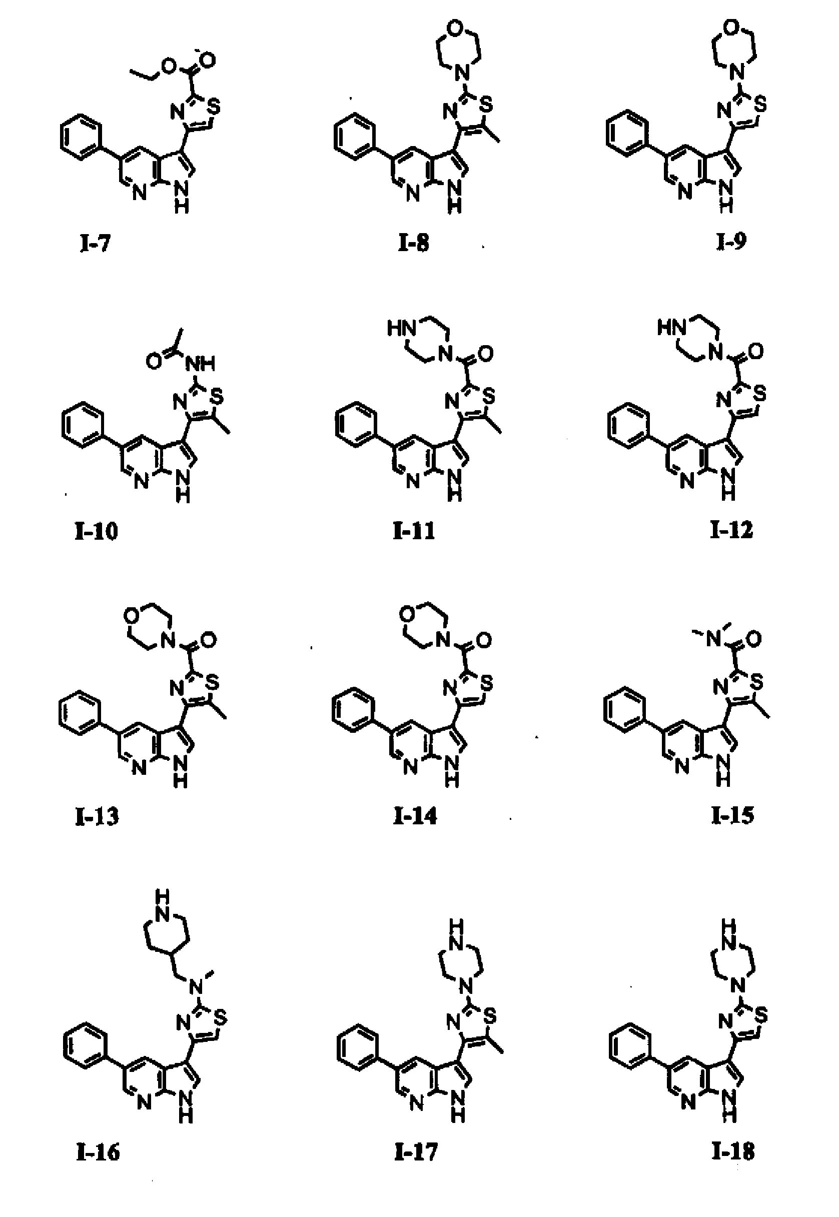

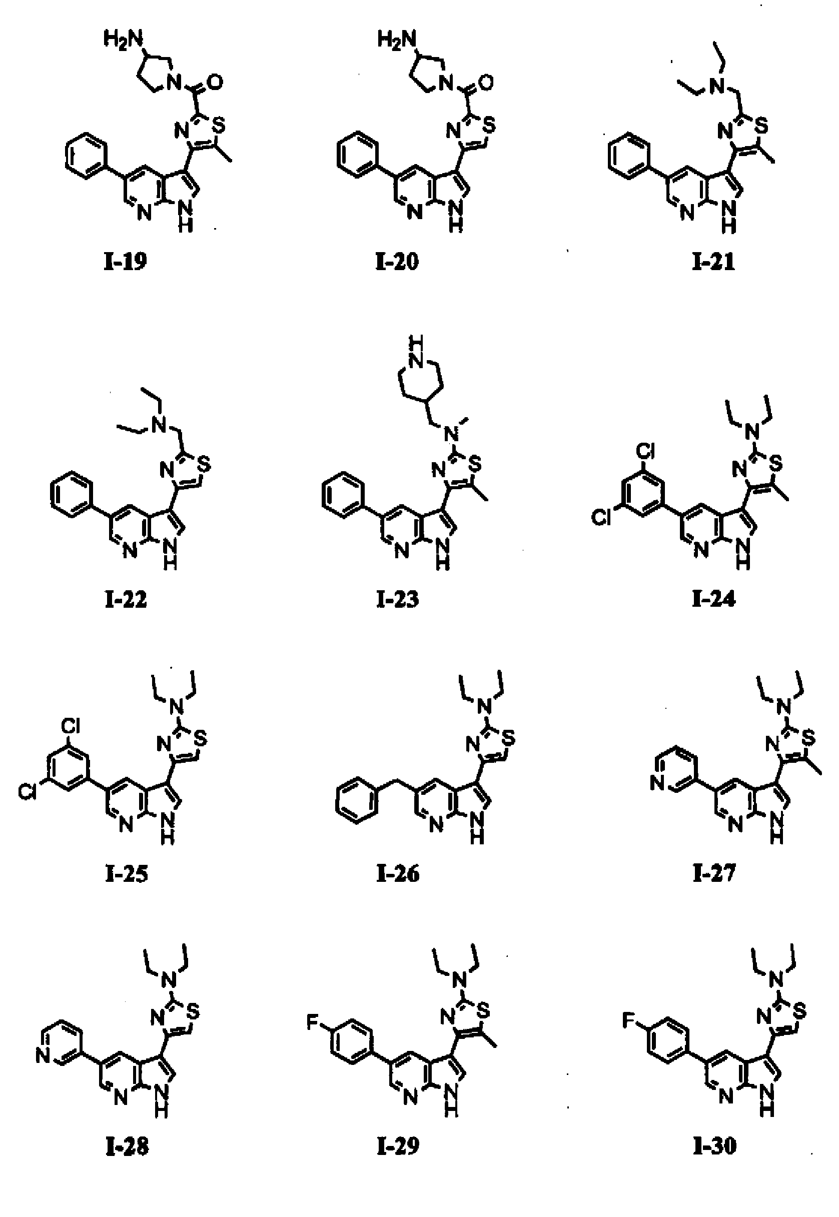

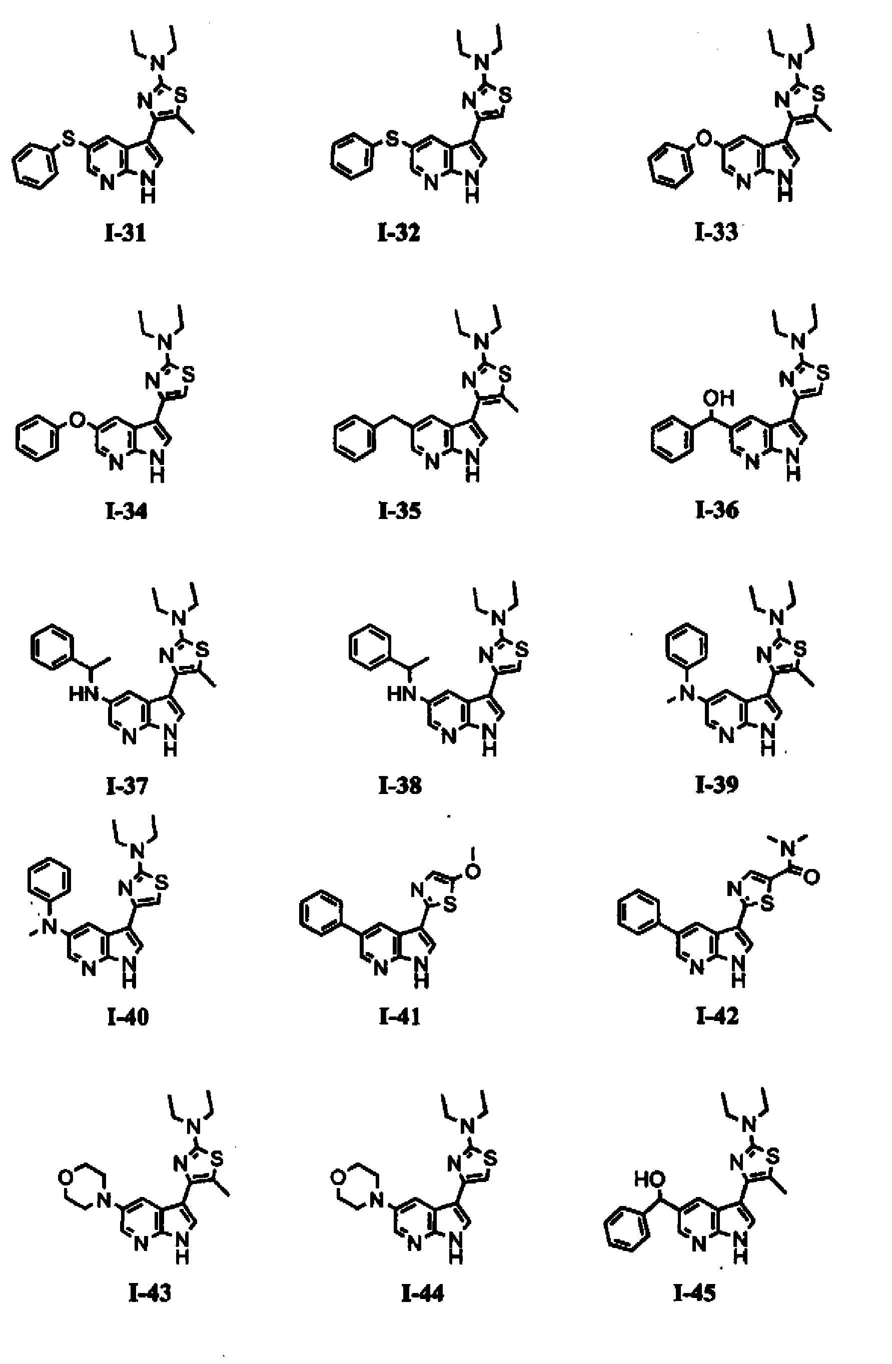

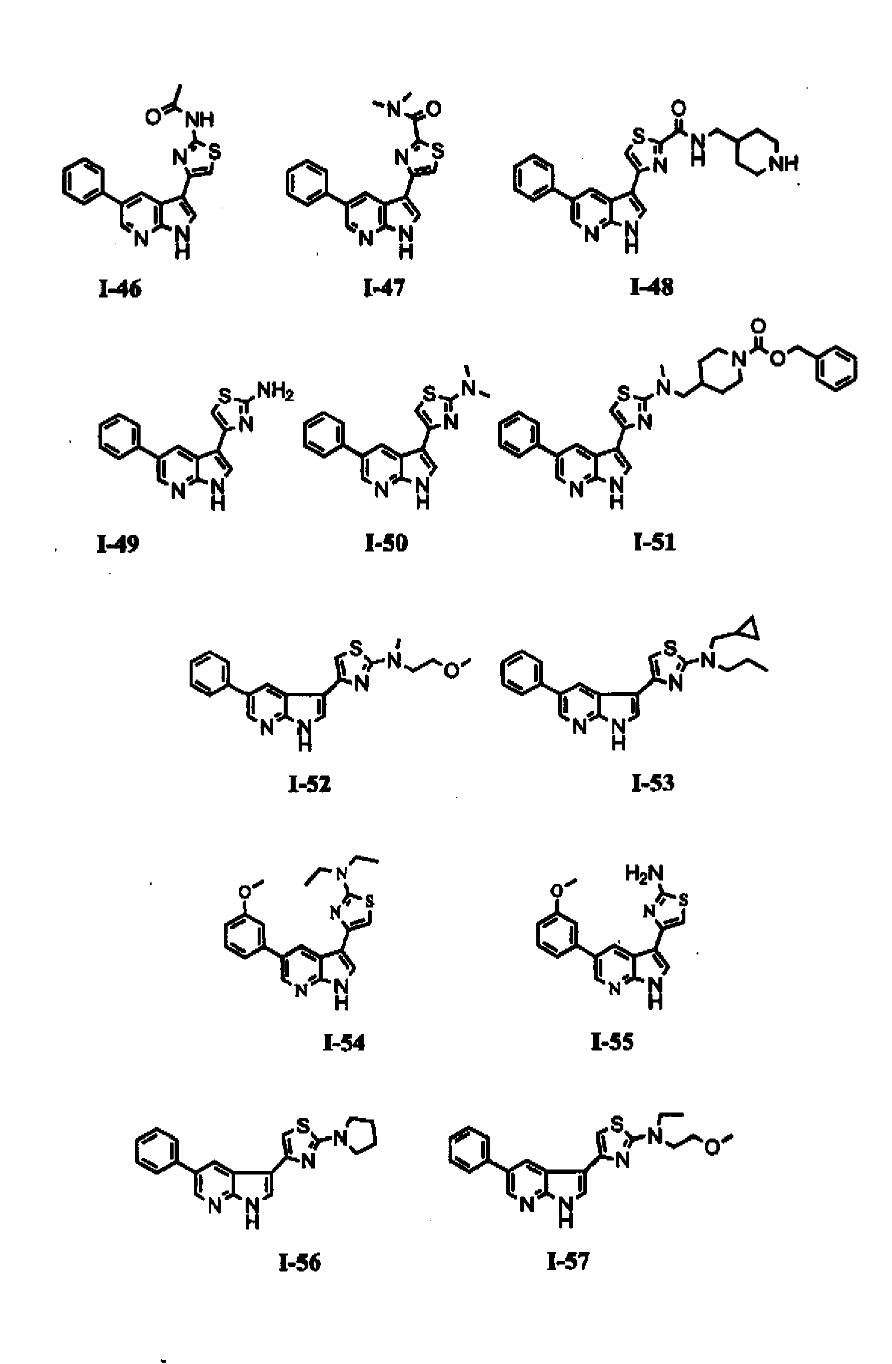

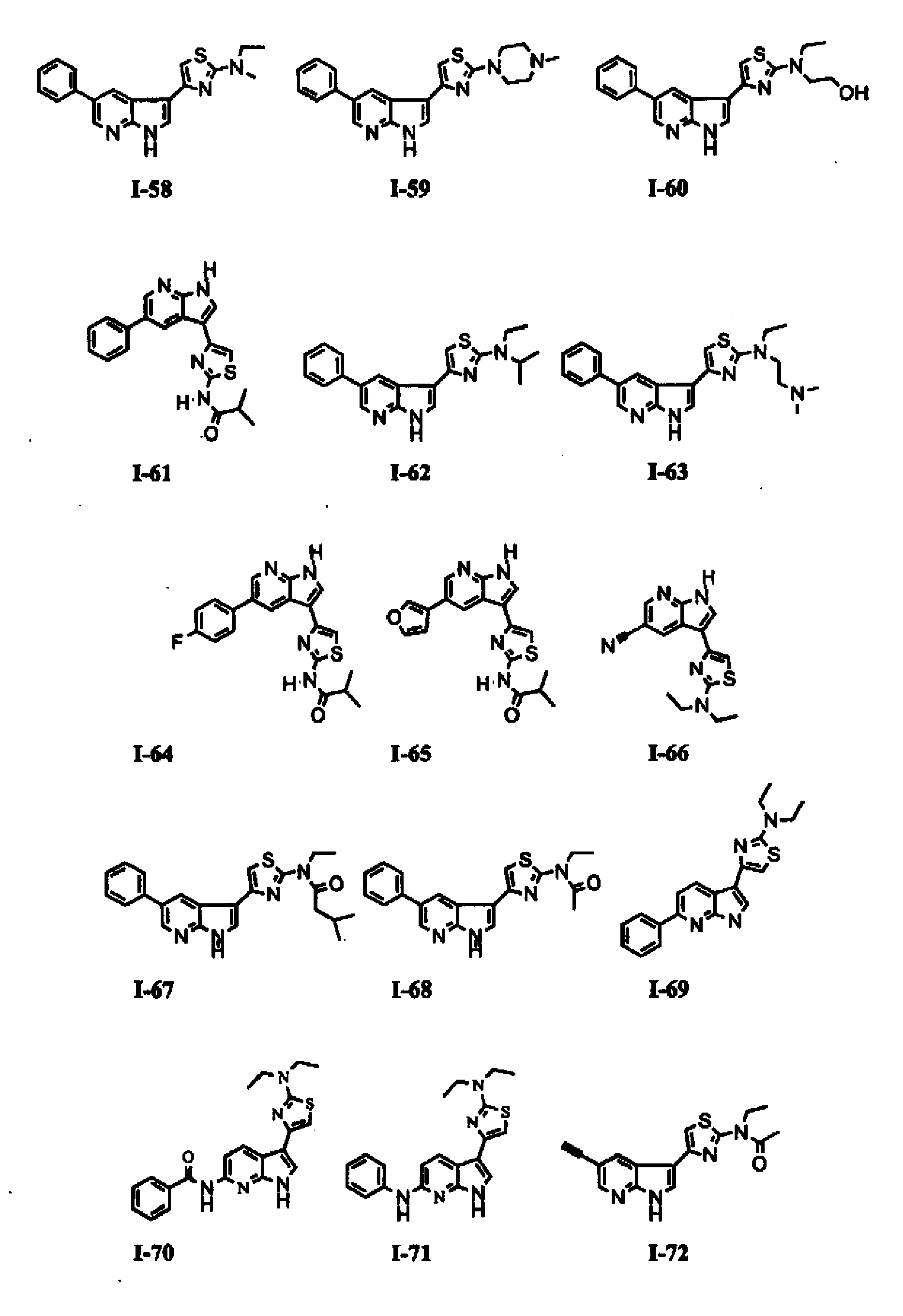

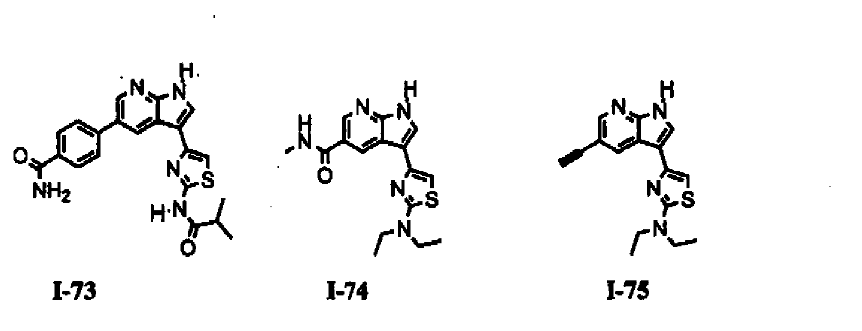

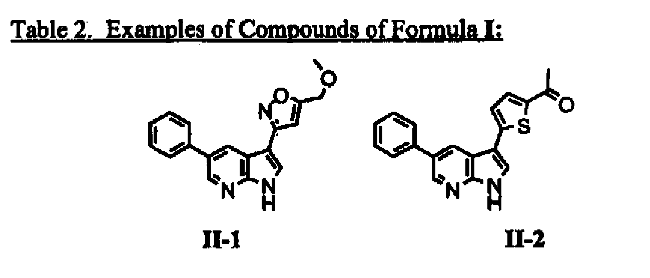

### 4. General Synthetic Methodology:

The compounds of this invention may be prepared in general by methods known to those skilled in the art for analogous compounds, as illustrated by the general scheme below, and the preparative examples that follow.

The following abbreviations are used: EtOH is ethanol
RT is room temperature
Ts is Tosyl
Ph is phenyl
DME is dimethylether
Bu is butyl
EDC is 1-Ethyl-3-(3-dimethyllaminopropyl)carbodiimide
DMF is dimethylformamide
O/N is overnight
Et₂O is ether
CDI is N,N'-Carbonyldiimidazole
LCMS liquid chromatography mass spectrometry
P is a suitable protecting group

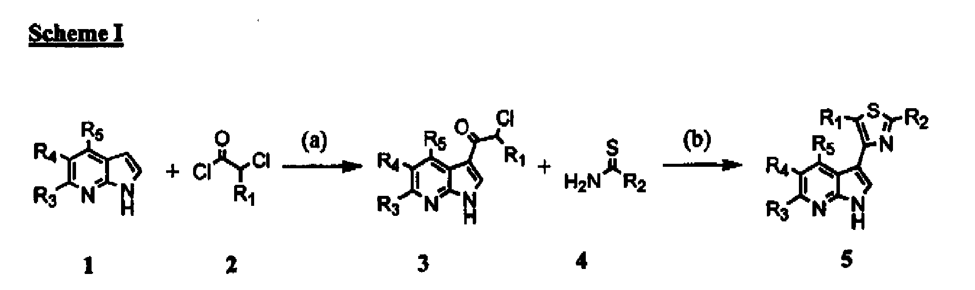

Reagents and conditions: (a) AlCl₃, CH₂Cl, RT, 16 hours; (b) EtOH, microwave irradiations, 120°C, 10 mins.

Scheme I above shows a general synthetic route that is used for preparing the compounds **5** of this invention when R₁ to R₅ are as described herein. Intermediates **3** arc prepared by using the Friedel-Craft acylation methods that are well known in the art. This reaction is amenable to a variety of substituted chloroacetyl chlorides to form compounds of formula **3**. Finally, compound of formula **5** is obtained by cyclisation of intermediate **3** according to step (b). The reaction is amenable to a variety of substituted thioamides of formula **4**.

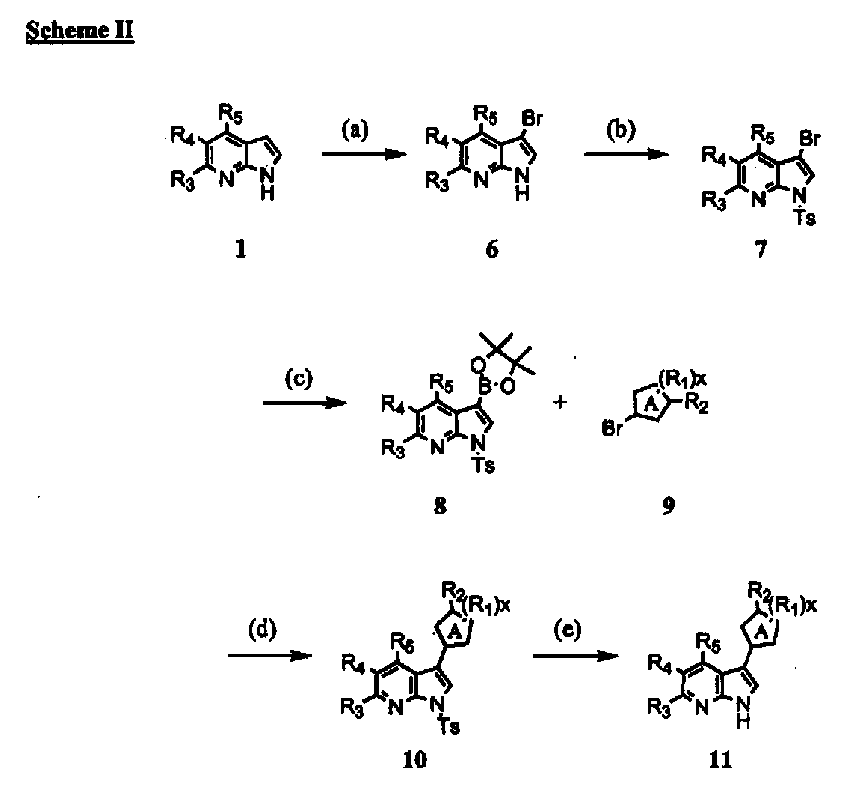

Reagents and conditions: (a) Br₂, CHCl₃, 0°C to RT; (b) "BuLi, THF, TsCl; (c) PdCl₂(dppf)₂, dioxane, KOAc, bis(pinacolato)diboron, 18 hours; (d) Pd(PPh₃)₄, Na₂CO₃, DME, EtOH/H₂O, microwave irradiation, 120°C, 2 hours; (e) 3N NaOH, MeOH.

Scheme II above shows a general synthetic route that is used for preparing the compounds **11** this invention when A, R₁ to R₅ and x are as described herein. Intermediate **7** is prepared by bromination of compound of structure **1** followed by subsequent protection of intermediate **6** with a tosyl group. Boronic esters **8** are formed according to Scheme II step (c). The formation of the biaryl link derivatives **10** is achieved by treating the bromide 9 with boronic ester derivatives **8** in the presence of palladium as a catalyst by using the Suzuki coupling methods that are well known in the art. The reaction is amenable to a variety of substituted aryl or heteroaryl bromides **9**. Finally, the tosyl protective group is removed in basic conditions, according to Scheme II step (e), to afford compounds of structure **11**.

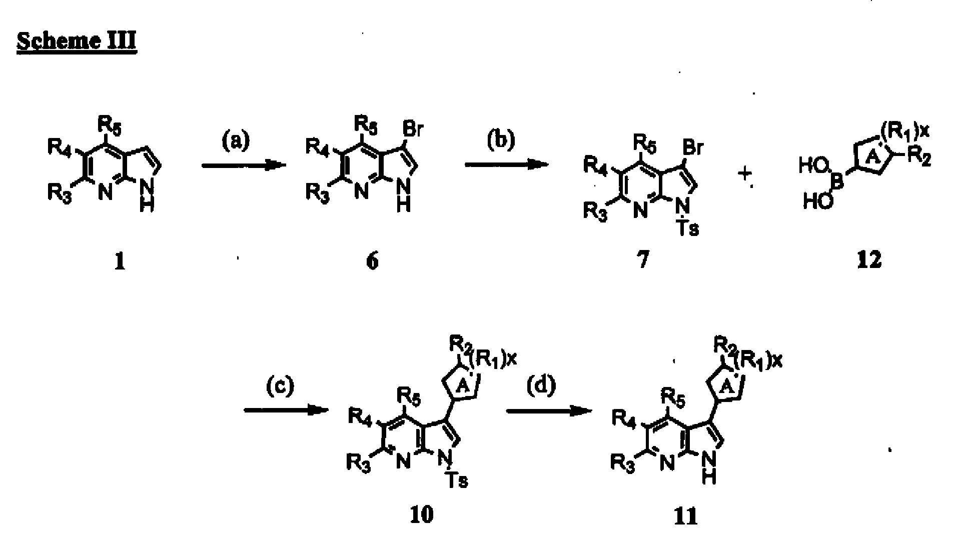

Reagents and conditions: (a) Br₂, CHCl₃, 0°C to RT; (b) "BuLi, THF, TsCl; (c) Pd(PPh₃)₄, Na₂CO₃, DME, EtOH/H₂O, microwave irradiations, 120°C, 2 hours; (d) 3N NaOH, MeOH.

Scheme III above shows another general synthetic route that has been used for preparing compounds **11** of this invention when A, R₁ to R₅ and x are as described herein. Intermediate **7** is prepared as above according to Scheme II. In this case, the formation of the biaryl link derivatives **10** is achieved by treating bromides **7** with a boronic acid derivative **12** in the presence of palladium as a catalyst by using the Suzuki coupling methods that are well known in the art. The reaction is amenable to a variety of substituted aryl or heteroaryl boronic acids **12**. Once again, the tosyl protective group is removed in basic conditions, according to Scheme III step (d), to afford compounds of structure **11.**

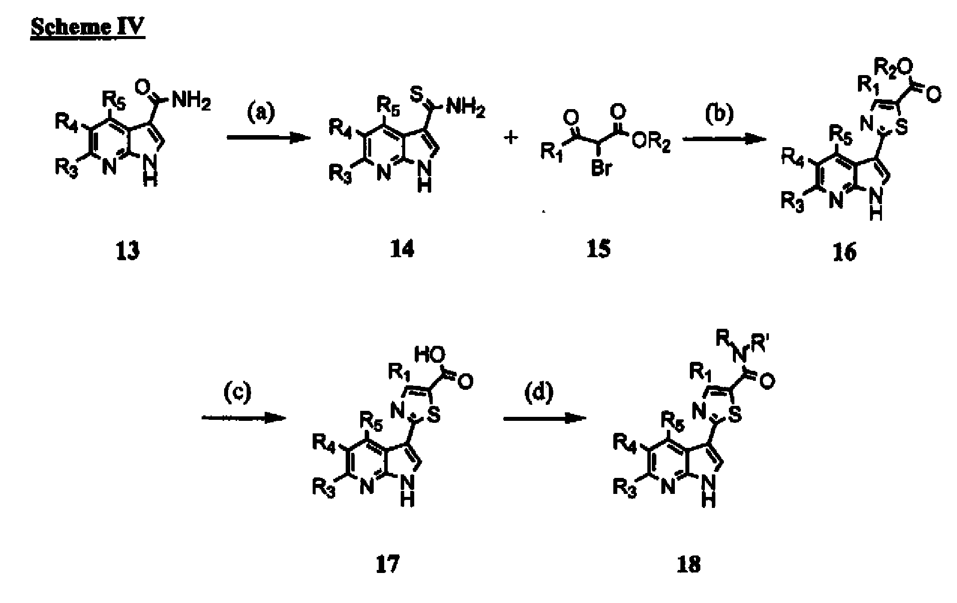

Reagents and conditions: (a) Lawesson's reagent, Toluene, 110°C, O/N; (b) EtOH, reflux, O/N; (c) EtOH, 1N NaOH, **12** hours; (d) EDC, HOBt, DMF, NHR'R, RT, OIN.

Scheme IV above shows a general synthetic route that has been used for preparing compounds **18** of this invention when R, R' and R₁ to R₅ are as described herein. Starting materials **13** may be prepared by methods substantially similar to those described in the literature by Schneller and Luo J. Org. Chem. 1980, 45, 4045. Derivatives **14** are formed by reaction of compounds **13** with Lawesson's reagent. The cyclisation of compounds **14** in presence of β-ketoesters **15** afford intermediates 16. The reaction is amenable to a variety of β-ketoesters **15.** After deprotection of the esters **16** under basic conditions, derivatives **18** are formed by a coupling reaction step well known to one of skill in the art.

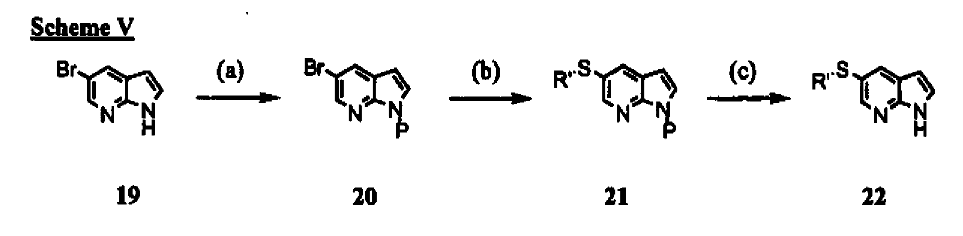

Regents and conditions: (a) "BuLi, THF, PCI; (b) i) 'BuLi, Et2O, -78°C, 1h, ii) R'SSR'; (c) Deprotection conditions.

Scheme V above shows a general synthetic route that has been used for preparing compounds **18** of this invention when R' is as described herein. Starting material **19** may be prepared by methods described by Mazeas, et al, Heterocycles 1999, 50, 1065. Intermediate **20**, obtained by protection of **19** with a suitable protecting group (P), is treated with the appropriate disulfide R'SSR' according to Scheme V step (b). After deprotection of the indazole **21**, compounds of formula **22** are formed.

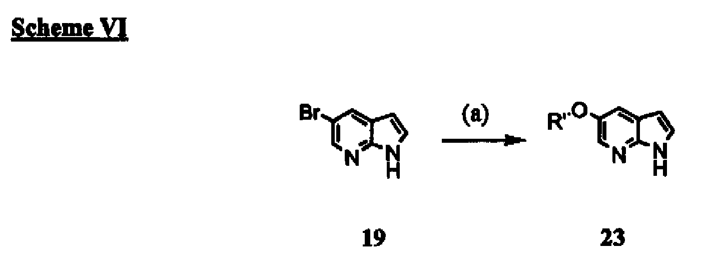

Reagents and conditions: (a) R₁ OH, NaOMe, CuBr, DMF, Heating, 2.5 hours.

Scheme VI above shows a general synthetic route that has been used for preparing compounds **23** of this invention when R' is as described herein. Starting material **19** is treated with the appropriate alcohol R'OH according to Scheme VI step (a).

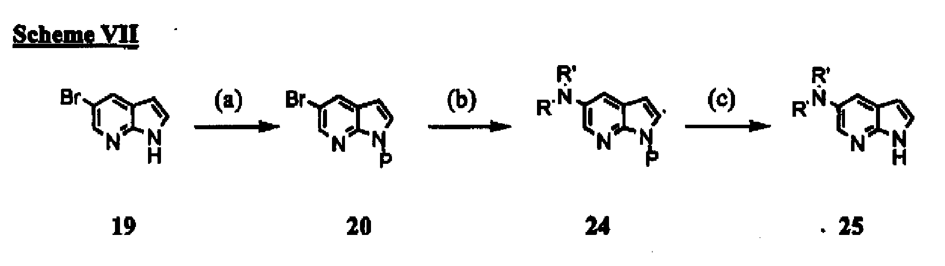

Reagents and conditions: (a) "BuLi, THF, PCI; (b) NHR'R, PdCl₂(dppf), NaO*^{t}*Bu, THF, heating; or HNR'R, Cu, K₂CO₃, nitrobenzene, heating; (c) Deprotection conditions.

Scheme VII above shows a general synthetic route that has been used for preparing compounds **25** of this invention when R and R' are as described herein. Intermediate **20,** obtained by protection of **19** with a suitable protecting group (P), is treated with an amine RR'NH in the presence of palladium as a catalyst by using the Buchwald-Hartwig cross coupling reaction well known in the art. This cross coupling reaction could also be achieved by treating intermediate **20** with an amine RR'NH in the presence of copper as a catalyst by using the Ullmann reaction well known in the art. Both these reactions are amenable to a variety of substituted amines. After deprotection of the indazole **24**, compounds of formula **25** are formed.

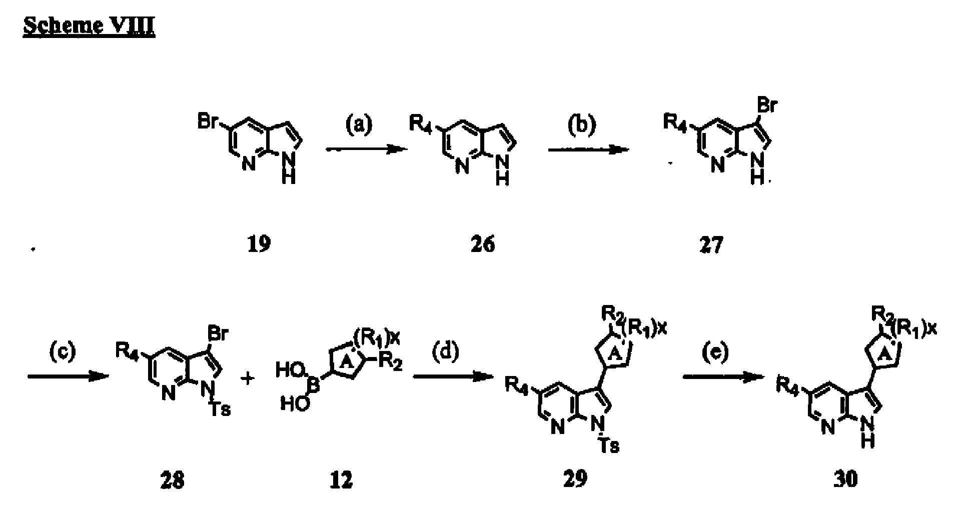

Reagents and conditions: (a) R₄B(OH)₂, Pd(PPh₃)₄, EtOH, H₂O, DME, 100°C, O/N; (b) Br₂, CHCl₃, 0°C to RT; (c) "BuLi, THF, TsCl; (d) Pd(PPh₃)₄, Na₂CO₃, DME, EtOH/H₂O, microwave irradiation, 120°C, 2 hours; (e) 3N NaOH, MeOH.

Scheme VIII above shows a general synthetic route that has been used for preparing compounds **30** of this invention when A, R₁ to R₄ and x are as described herein. Compound of structure **19** is treated with a boronic acid derivative R₄B(OH)₂ in the presence of palladium as a catalyst by using the Suzuki coupling method which is well known in the art. The reaction is amenable to a variety of substituted aryl or heteroaryl boronic acids. Intermediate **27** is prepared by bromination of compounds of structure **26** followed by subsequent protection of intermediate **27** with a tosyl group. Another Suzuki cross coupling reaction is achieved according to Scheme VIII step (d). Finally, the tosyl protective group is removed in basic conditions, according to Scheme VIII step (e), to afford compounds of structure **30**.

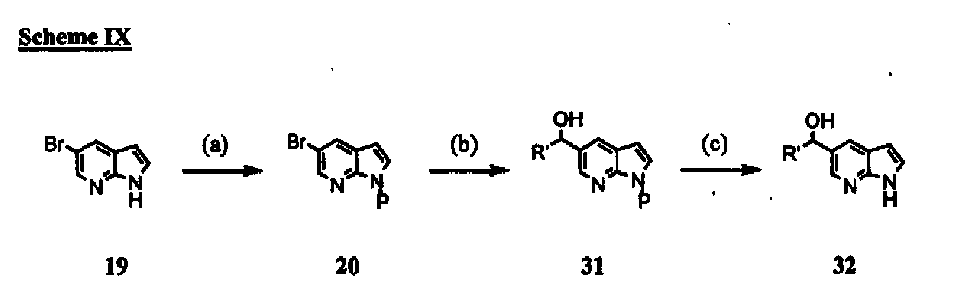

Reagents and conditions: (a) "BuLi, THF, PCI; (b) i) 'BuLi, Et₂O, -78°C, 1h, ii) R'CHO; (c) Deprotection conditions.

Scheme IX above shows a general synthetic route that has been used for preparing compounds **32** of this invention when R' is as described herein. Intermediate **20**, obtained by protection of **19** with a suitable protecting group (P), is treated with the appropriate aldehyde R'CHO according to Scheme IX step (b). After deprotection of the indazole **31**, compounds of formula **32** are formed.

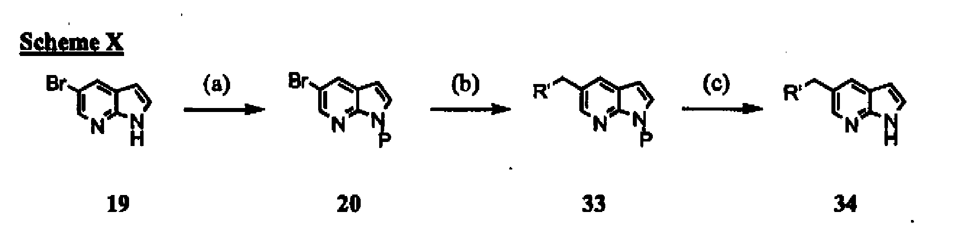

Reagents and conditions: (a) "BuLi, THF, PCI; (b) i) *^{t}*BuLi, Et2O, -78°C, 1h, ii) R'CH₂Br; (c) Deprotection conditions.

Scheme X above shows a general synthetic route that has been used for preparing compounds **32** of this invention when R' is as described herein. Intermediate **20**, obtained by protection of **19** with a suitable protecting group (P), is treated with the appropriate R'CH₂Br according to Scheme X step (b). After deprotection of the indazole **33**, compounds of formula **34** are formed.

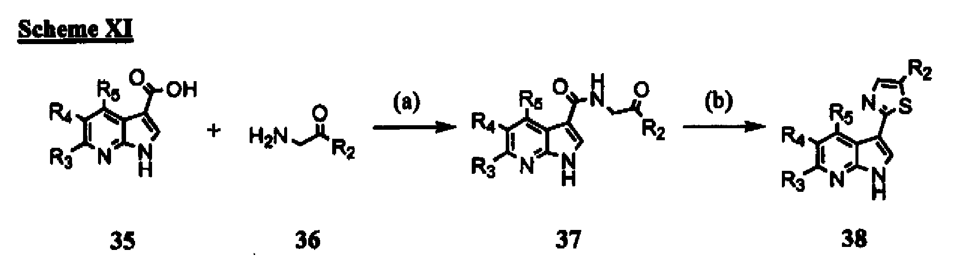

Reagents and conditions: (a) CDI, DMF; (b) P₂S₅, pyridine.

Scheme XI above shows a general synthetic route that has been used for preparing compounds **38** of this invention when R₂ to R₅ are as described herein. Starting materials **35** may be prepared by methods substantially similar to those described in the literature by Allegreti et al, Org. Proc. Res. Dev. 2003, 7, 209. Intermediates **35** react with amines **36** following Scheme XI step (a). The reaction is amenable to a variety of amines **36**. The cyclisation of compounds **37** in presence of P₂S₅ affords the desired derivatives **38**.

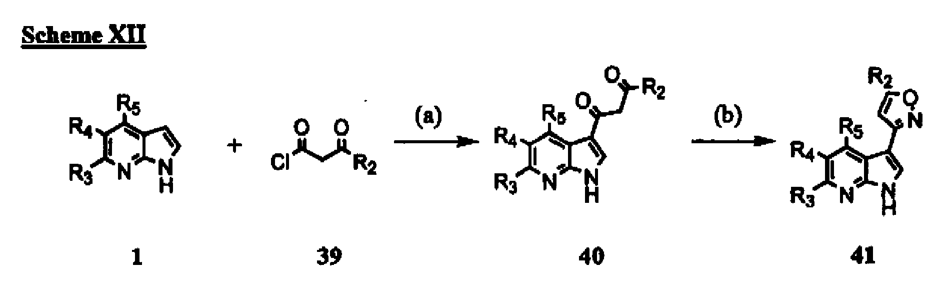

Reagents and conditions: (a) AlCl₃, CH₂Cl₂, RT, 16 hours; (b) NH₂OH.HCl, EtOH, heating, 1 hour.

Scheme XII above shows a general synthetic route that has been used for preparing compounds **41** of this invention when R₂ to R₅ are as described herein. Intermediates **40** are prepared by using the Friedel-Craft acylation methods that are welt known in the art. This reaction is amenable to a variety of substituted derivatives **39** to form compounds of formula **40**. Compounds of formula **41** are obtained by cyclisation of intermediate **40** according to step (b).

Although certain exemplary embodiments are depicted and described above and herein, it will be appreciated that a compounds of the invention can be prepared according to the methods described generally above using appropriate starting materials by methods generally available to one of ordinary skill in the art.

Accordingly, in another embodiment, this invention provides processes for preparing a compound of this invention substantially as described herein and particularly as described in the Schemes and Examples.

### 5. Uses, Formulation and Administration

### Pharmaceutically acceptable compositions

As discussed above, the present invention provides compounds that are inhibitors of protein kinases, and thus the present compounds are useful for the treatment of diseases, disorders, and conditions including, but not limited to an autoimmune, inflammatory, proliferative, or hyperproliferative disease or an immunologically-mediated disease. Accordingly, in another aspect of the present invention, pharmaceutically acceptable compositions are provided, wherein these compositions comprise any of the compounds as described herein, and optionally comprise a pharmaceutically acceptable carrier, adjuvant or vehicle. In certain embodiments, these compositions optionally further comprise one or more additional therapeutic agents.

It will also be appreciated that certain of the compounds of present invention can exist in free form for treatment, or where appropriate, as a pharmaceutically acceptable derivative thereof. According to the present invention, a pharmaceutically acceptable derivative includes, but is not limited to, pharmaceutically acceptable salts, esters, salts of such esters, or any other adduct or derivative which upon administration to a patient in need is-capable of providing, directly or indirectly, a compound as otherwise described herein, or a metabolite or residue thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of a compound of this invention that, upon administration to a recipient, is capable of providing, either directly or indirectly, a compound of this invention or an inhibitorily active metabolite or residue thereof. As used herein, the term "inhibitorily active metabolite or residue thereof" means that a metabolite or residue thereof is also an inhibitor of a Tec family (e.g.,Tec, Btk, Itk/Emt/Tsk, Bmx, Txk/Rlk) protein kinases kinase.

Pharmaceutically acceptable salts are well known in the art. For example, S. M. Berge *et al.,* describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄alkyl)₄ salts. This invention also envisions the quaternization of any basic nitrogen-containing groups of the compounds disclosed herein. Water or oil-soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As described above, the pharmaceutically acceptable compositions of the present invention additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogon-free water; isotonic saline; Ringer's solution; ethyl alcohol, and phosphate buffer solutions, as well as other non toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

### Uses of Compounds and Pharmaceutically acceptable compositions

In yet another aspect, a method for the treatment or lessening the severity of a Tec family (e.g.,Tec, Btk, Itk/Emt/Tsk, Bmx, Txk/Rlk)-mediated diseases is provided comprising administering an effective amount of a compound, or a pharmaceutically acceptable composition comprising a compound to a subject in need thereof. In certain embodiments of the present invention an "effective amount" of the compound or pharmaceutically acceptable composition is that amount effective for a Tec family (e.g.,Tec, Btk, Itk/Emt/Tsk, Bmx, Txk/Rlk)-mediated disease. The compounds and compositions, according to the method of the present invention, may be administered using any amount and any route of administration effective for treating or lessening the severity of a Tec family (e.g.,Tec, Btk, Itk/Emt/Tsk, Bmx, Txk/Rlk)-mediated disease. The exact amount required will vary from subject to subject, depending on the species, age, and general condition of the subject, the severity of the infection, the particular agent, its mode of administration, and the like. The compounds of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient", as used herein, means an animal, preferably a mammal, and most preferably a human.

The pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparation, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

As described generally above, the compounds of the invention are useful as inhibitors of protein kinases. In one embodiment, the compounds and compositions of the invention are inhibitors of one or more of Tec family (e.g.,Tec, Btk, Itk/Emt/Tsk, Bmx, Txk/Rlk) kinase, and thus, without wishing to be bound by any particular theory, the compounds and compositions are particularly useful for treating or lessening the severity of a disease, condition, or disorder where activation of one or more of a Tec family (e.g.,Tec, Btk, Itk/Emt/Tsk, Bmx, Txk/Rlk) kinase is implicated in the disease, condition, or disorder. When activation of Tec family (e.g.,Tec, Btk, Itk/Emt/Tsk, Bmx, Txk/Rlk) is implicated in a particular disease, condition, or disorder, the disease, condition, or disorder may also be referred to as a "Tec family (e.g.,Tec, Btk, Itk/Emt/Tsk, Bmx, Txk/Rlk)-mediated disease" or disease symptom. Accordingly, in another aspect, the present invention provides a method for treating or lessening the severity of a disease, condition, or disorder where activation or one or more of Tec family (e.g.,Tec, Btk, Itk/Emt/Tsk, Bmx, Txk/Rlk) is implicated in the disease state.

The activity of a compound utilized in this invention as an inhibitor of a Tec family (e.g.,Tec, Btk, Itk/Emt/Tsk, Bmx, Txk/Rlk) kinase may be assayed *in vitro, in vivo* or in a cell line. *In vitro* assays include assays that determine inhibition of either the phosphorylation activity or ATPase activity of activated Tec family (e.g.,Tec, Btk, Itk/Emt/Tsk, Bmx, Txk/Rlk) kinase. Alternate *in vitro* assays quantitate the ability of the inhibitor to bind to a Tec family (e.g.,Tec, Btk, Itk/Emt/Tsk, Bmx, Txk/Rlk) kinase. Inhibitor binding may be measured by radiolabelling the inhibitor prior to binding, isolating the inhibitor/Tec family (e.g.,Tec, Btk, Itk/Emt/Tsk, Bmx, Txk/Rlk), complex and determining the amount of radiolabel bound. Alternatively, inhibitor binding may be determined by running a competition experiment where new inhibitors are incubated with a Tec family (e.g.,Tec, Btk, Itk/Emt/Tsk, Bmx, Txk/Rlk) kinase bound to known radioligands.

The term "measurably inhibit", as used herein means a measurable change in a Tec family (e.g.,Tec, Btk, Itk/Emt/Tsk, Bmx, Txk/Rlk) kinase activity between a sample comprising said composition and a Tec family (e.g.,Tec, Btk, Itk/Emt/Tsk, Bmx, Txk/Rlk) kinase and an equivalent sample comprising a Tec family (e.g.,Tec, Btk, Itk/Emt/Tsk, Bmx, Txk/Rlk) kinase in the absence of said composition.

The term "Tec family tyrosine kinases-mediated condition", as used herein means any disease or other deleterious condition in which Tec family kinases are known to play a role. Such conditions include, without limitation, autoimmune, inflammatory, proliferative, and hyperproliferative diseases and immunologically-mediated diseases including rejection of transplanted organs or tissues and Acquired Immunodeficiency Syndrome (AIDS).

For example, Tec family tyrosine kinases -mediated conditions include diseases of the respiratory tract including, without limitation, reversible obstructive airways diseases including asthma, such as bronchial, allergic, intrinsic, extrinsic and dust asthma, particularly chronic or inveterate asthma (e.g. late asthma airways hyper-responsiveness) and bronchitis. Additionally, Tec family tyrosine kinases diseases include, without limitation, those conditions characterised by inflammation of the nasal mucus membrane, including acute rhinitis, allergic, atrophic thinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis, seasonal rhinitis including rhinitis nervosa (hay fever) and vasomotor rhinitis, sarcoidosis, farmer's lung and related diseases, fibroid lung and idiopathic interstitial pneumonia.

Tec family tyrosine kinases -mediated conditions also include diseases of the bone and joints including, without limitation, (pannus formation in) rheumatoid arthritis, seronegative spondyloarthropathis (including ankylosing spondylitis, psoriatic arthritis and Reiter's disease), Behcet's disease, Sjogren's syndrome, and systemic sclerosis.

Tec family kinases-mediated conditions also include diseases and disorders of the skin, including, without limitation, psoriasis, systemic sclerosis, atopical dermatitis, contact dermatitis and other eczematous dermatitis, seborrhoetic dermatitis, Lichen planus, Pemphigus, bullous Pemphigus, epidermolysis bullosa, urticaria, angiodermas, vasculitides, erythemas, cutaneous eosinophilias, uveitis, Alopecia, areata and vernal conjunctivitis.

Tec family tyrosine kinases-mediated conditions also include diseases and disorders of the gastrointestinal tract, including, without limitation, Coeliac disease, proctitis, eosinophilic gastro-enteritis, mastocytosis, pancreatitis, Crohn's disease, ulcerative colitis, food-related allergies which have effects remote from the gut, e.g. migraine, rhinitis and eczema.

Tec family tyrosine kinases-mediated conditions also include those diseases and disorders of other tissues and systemic disease, including, without limitation, multiple sclerosis, artherosclerosis, acquired immunodeficiency syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia purpura, restenosis following angioplasty, tumours (for example leukemia, lymphomas), artherosclerosis, and systemic lupus erythematosus.

Tec family tyrosine kinases-mediated conditions also include allograft rejection including, without limitation, acute and chronic allograft rejection following for example transplantation of kidney, heart, liver, lung, bone marrow, skin and cornea; and chronic graft versus host disease.

It will also be appreciated that the compounds and pharmaceutically acceptable compositions of the present invention can be employed in combination therapies, that is, the compounds and pharmaceutically acceptable compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated".

For example, chemotherapeutic agents or other anti-proliferative agents may be combined with the compounds of this invention to treat proliferative diseases and cancer. Examples of known chemotherapeutic agents include, but are not limited to, For example, other therapies or anticancer agents that may be used in combination with the inventive anticancer agents of the present invention include surgery, radiotherapy (in but a few examples, gamma.-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), Gleevec™, adriamycin, dexamethasone, and cyclophosphamide. For a more comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999, the entire contents of which are hereby incorporated by reference.

Other examples of agents the inhibitors of this invention may also be combined with include, without limitation: treatments for Alzheimer's Disease such as Aricept^{®} and Excelon^{®}; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; antiinflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophonolate mofetil, interferons, corticosteroids, cyclophosphamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

The compounds of this invention or pharmaceutically acceptable compositions thereof may also be incorporated into compositions for coating implantable medical devices, such as prostheses, artificial valves, vascular grafts, stents and catheters. Accordingly, the present invention, in another aspect, includes a composition for coating an implantable device comprising a compound of the present invention as described generally above, and in classes and subclasses herein, and a carrier suitable for coating said implantable device. In still another aspect, the present invention includes an implantable device coated with a composition comprising a compound of the present invention as described generally above, and in classes and subclasses herein, and a carrier suitable for coating said implantable device.

Vascular stents, for example, have been used to overcome restenosis (re-narrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

Another aspect of the invention relates to inhibiting Tec family (e.g.,Tec, Btk, Itk/Emt/Tsk, Bmx, Txk/Rlk) activity in a biological sample or a patient, which method comprises administering to the patient, or contacting said biological sample with a compound of formula I or a composition comprising said compound. The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition of Tec family (e.g.,Tec, Btk, Itk/Emt/Tsk, Bmx, Txk/Rlk) kinase activity in a biological sample is useful for a variety of purposes that are known to one of skill in the art. Examples of such purposes include, but are not limited to, blood transfusion, organ-transplantation, biological specimen storage, and biological assays.

### SYNTHETIC EXAMPLES

As used herein ¹H NMR is nuclear magnetic resonance. HPLC is high performance liquid chromatography. The term "Rt(min)" refers to the HPLC retention time, in minutes, associated with the compound. Unless otherwise indicated, the HPLC method utilized to obtain the reported retention time is as follows:
Column: Ace 5 C8, 15cm x 4.6mm id
Gradient: 0-100% acetonitrile+methanol (50:50) (20mM Tris phosphate at pH 7.0)
Flow rate: 1.5 ml/min
Detection: 225 nm
**Example 1**

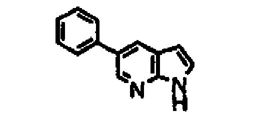

**5-Phenyl-1*H-*pyrrolo[2,3-*b*]pyridine**

5-Bromo-1*H-*pyrrolo[2,3-*b*]pyridine (2 g, 14.15 mmol), phenylboronic acid (1.24 g, 10.15 mmol) and tetrakis-(triphenylphosphine) palladium (117 mg, 0.10 mmol) were suspended in ethanol (5 ml), water (6 ml) and DME (22 ml) and heated to 100 °C overnight. The solvent were removed *in vacuo* and the reaction purified by column chromatography eluting with 30% ethyl acetate in petrol to give the *title compound* as an off-white solid (1.51 g, 77%). MS (ES⁺) 195, (ES⁻) 193. δH (CDCl₃) 6.60 (1H, s), 7.36-7.43 (2H, m), 7.68 (2H, d), 8.18 (1H, s), 8.62 (1H, s), 10.39 (1H, br s).
**Example 2**

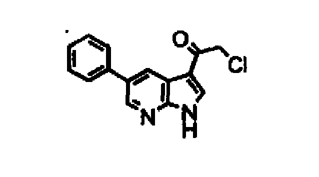

**2-Chloro-1-(5-phenyl-1*H-*pyrrolo[2,3*-b*]pyridin-3-yl)-ethanone** 5-Phenyl-1*H-*pyrrolo[2,3*-b*]pyridine (200 mg, 1.03 mmol) and aluminum chloride (412 mg, 3.09 mmol) were suspended in dry DCM and stirred at room temperature for 1 hour. chloroacetyl chloride (98 µl, 1.24 mmol) was added drop wise and the resulting amber solution was stirred at room temperature overnight. The reaction was quenched with methanol (5 ml) and stirred at room temperature for 2 hours. The solvent was then evaporated to give an orange oil. This was partitioned between DCM and water. The organic was concentrated in *vacuo* and the product triturated with diethyl ether to give the *title compound* as a beige solid (188 mg, 67%). MS (ES⁺) 271, (ES⁻) 269. δH (CDCl₃) 4.57 (2H, s), 7.44 (1H, t), 7.50 (2H, t), 7.70 (2H, d), 8.23 (1H, s), 8.70 (1H, s), 8.91 (1H, s), 11.59 (1H, br s).
**Example 3**

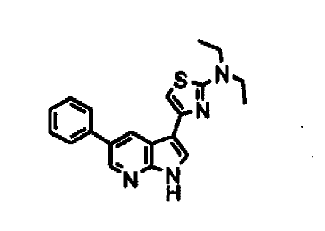

**Diethyl-[4-(5-phenyt-1*H-*pyrrolo[2,3*-b*]pyrridin-3-yl)-thiazol-2-yl]-amine**

2-Chloro-1-(5-phenyl-1*H-*pyrrolo[2,3*-b*]pyridin-3-yl)-ethanone (50 mg, 0.18 mmol) and 1,1-diethylthiourea (24 mg, 0.18 mmol) were suspended / dissolved in ethanol (2 ml) and heated in the microwave at 120 °C for 10 minutes. The crude reaction mixture was purified by HPLC eluting with acetonitrile water to give the tile compound as a cream-coloured solid (9.5 mg, 15%). MS (ES+) 349, (ES-) 347. δH (CDCl3) 1.31 (6H, t), 3.59 (4H, q), 6.61 (1H, s), 7.38 (1H, t), 7.50 (2H, t), 7.69 (2H, d), 7.78 (1H, s), 8.56-8.62 (2H, m), 8.94 (1H, br s).

A variety of other compounds of Formula I have been prepared by methods substantially similar to those described herein Example 3. The characterization data for these compounds is summarized in Table 3 below and includes HPLC, LC/MS (observed) and ¹H NMR data.

**Table 3. Characterization Data for Selected Compounds of Formula I**

| **Compound No I-** | **M+1(obs)** | **Rt (min)** | **¹H-NMR** |
|---|---|---|---|
| **1** | 292 | 9.60 | (CDCl₃) 2.82 (3H, s), 7.26 (1H, s), 7.40 (1H, t), 7.52 (2H, t), 7.70 (2H, d), 7.89 (1H, s), 8.53 (1H, s), 8.62 (1H, s), 9.07 (1H, br s) |
| **2** | 349 | 10.72 | (CDCl3) 1.31 (6H, t), 3.59 (4H, q), 6.61 (1H, s), 7.38 (1H, t), 7.50 (2H, t), 7.69 (2H, t), 7.78 (1H, s), 8.56-8.62 (2H, m), 8.94 (1H, br s) |
| **3** | 306 | 9.96 | (CDCl₃) 2.56 (3H, s), 2.75 (3H, s), 7.39 (1H, t), 7.46-7.53 (3H, m), 7.68 (2H, d), 8.58 (1H, s), 8.61 (1H, s), 9.44 (1H, br s) |
| **4** | 363 | 10.98 | (CDCl₃) 1.33 (6H, t), 2.47 (3H, s), 3.54 (4H, q), 7.36 (1H, t), 7.45-7.51 (3H, m), 7.69 (2H, d), 8.60 (1H, s), 8.76 (1H, s), 9.14 (1H, br s) |
| **5** | 307 | 8.74 | (CDCl₃) 2.40 (3H, s), 5.02 (2H, s), 7.34-7.39 (2H, m), 7.46 (2H, t), 7.65 (2H, d), 8.51 (1H, s), 8.59 (1H, s), 9.96 (1H, br s) |
| **6** | 364 | 10.17 | (CDCl₃) 1.48 (3H, t), 2.70 (3H, s), 4.50 (2H, q), 7.39 (1H, t), 7.48 (2H, t), 7.61 (1H, s), 7.70 (2H, d), 8.65 (2H, s), 9.07 (1H, br s) |
| **7** | 350 | 9.89 | (CDCl₃) 1.50 (3H, t), 4.54 (2H, q), 7.41 (1H, t), 7.52 (2H, t), 7.66-7.72 (3H, m), 8.03 (1H, s), 8.55 (1H, s), 8.65 (1H, s), 9.26 (1H, br s) |
| **9** | 363 | 9.78 | (CDCl₃) 3.55-3.63 (4H, m), 3.86-3.92 (4H, m), 6.76 (1H, s), 7.36-7.44 (1H, m), 7.46-7.54 (1H, m), 7.83 (1H, s), 8.53 (1H, s), 8.59 (1H, s), 9.64 (1H, s) |
| **12** | 390 | .63 | (DMSO) 3.25-3.45 (4H, m), 3.90 (2H, br s), 4.76 (2H, br s), 7.42 (1H, t), 7.54 (2H, t), 7.79 (2H, d), 8.20 (1H, s), 8.36 (1H, s), 8.63 (2H, d), 8.82 (1H, br s), 12.20 (1H, s). |
| **14** | 391 | 9.20 | (CDCl₃) 3.81-3.90 (4H, m), 4.66 (2H, br s), 7.41 (1H, t), 7.54 (2H, t), 7.62-7.69 (3H, m), 7.84 (1H, s), 8.55 (1H, s), 8.65 (1H, s), 9.11 (1H, br s). |
| **16** | 404 | 8.89 | (CDCl₃) 1.18-1.34 (2H, m), 1.73-1.82 (2H, m), 1.98-2.37 (3H, m), 2.58-2.70 (2H, m), 3.20 (3H, s), 3.42-3.53 (2H, m), 6.62 (1H, s), 7.33-7.42 (1H, m), 7.45-7.54 (2H, m), 7.65-7.71 (2H, m), 7.80 (1H, s), 8.54-8.59 (2H, m), 9.42 (1H, br s) |
| **18** | 362 | | (DMSO) 2.81-2.87 (4H, m), 3.35-3.44 (4H, m), 7.27 (1H, s), 7.37 (1H, t), 7.45-7.52 (2H, m), 7.73-7.77 (2H, m), 7.84 (1H, s), 8.54 (1H, s), 8.59 (1H, s), 11.88 (1H, s) |
| **46** | 335 | 8.92 | (DMSO) 2.18 (3H, s), 7.43 (1H, t), 7.49-7.56 (3H, m), 4.79-4.82 (2H, m), 7.94 (1H, s), 8.58 (1H, s), 8.71 (1H, s), 12.00 (1H, s), 12.19 (1H, s) |
| **48** | 418 | 8.48 | (DMSO): 1.40 (2H, t), 1.80-1.95 (3H, m), 2.88 (2H, t), 3.22-3.31 (4H, m), 7.39 (1H, t), 7.51 (2H, t), 7.85 (2H, d), 8.15 (1H, s), 8.29 (2H, br s), 8.60 (1H, s), 8.78 (1H, s), 8.99 (1H, t), 12.17 (1H, s). |
| **49** | 293 | | (DMSO) 6.95 (1H, s), 7.00 (1H, s), 7.37 (1H, t), 7.46-7.51 (2H, m), 7.75-7.79 (3H, m), 8.54 (1H, s), 8.59 (1H, s), 11.84 (1H, s) |
| **50** | 321 | 10.00 | (DMSO) 3.13 (6H, s), 7.10 (1H, s), 7.39 (1H, t), 7.46-7.54 (2H, m), 7.75-7.78 (2H, m), 7.85 (1H, s), 8.54 (1H, s), 8.62 (1H, s), 11.88 (1H, s) |
| **50** | 538 | 10.9 | (CDCl₃) 1.60-1.80 (4H, m), 2.03-2.16 (1H, m), 2.73-2.83 (2H, m), 3.21 (3H, s), 3.2-3.50 (2H, m), 4.10-4,32 (2H, m), 5.15 (2H, s), 6.65 (1H, s), 7.26-7.40 (6H, m), 7.44-7.55 (2H, m), 7.65-7.70 (2H, m), 7.80 (1H, s), 8.59 (2H, s), 9.20 (1H, s) |
| **52** | 365 | 10.0 | (CDCl₃) 3.25 (3H, s), 3.44 (3H, s), 3.68-3.74 (2H, m), 3.75.3.80 (2H, m), 6.65 (1H, s), 7.36-7.44 (1H, m), 7.46-7.54 (2H, m), 7.65-7.72 (2H, m), 7.84 (1H, s), 8.58 (1H, s), 8.61 (1H, s), 9.51 (1H, br s) |
| **53** | 389 | 11.22 | (CDCl₃) 0.28-0.40 (2H, m), 0.53-0.63 (2H, m), 0,93-1.02 (3H, m), 1.15-1.26 (1H, m), 1.72-1.85 (2H, m), 3.38-3.45 (2H, m), 3.45-3.55 (2H, m), 6.61 (1H, s), 7.32-7.42 (1H, m), 7.42-7.53 (2H, m), 7.62-7.72 (2H, m), 7.78 (1H, s), 8.60 (1H, s), 8.66 (1H, s), 9.21 (1H, br s) |
| **54** | 379 | 10.6 | (DMSO) 1.2-1.3(6H, m), 3.4-3.6(4H, m), 3.8-3.9(3H, s), 6.9(1H, m), 7.0(1H, s), 7.3(2H, m), 7.4(1H, m), 7.8-7.9(1H, s), 8.5-8.6(1H, s), 8.7(1H, s), 11.8-11.9(0.7H, s) |
| **55** | 323 | 8.5 | (DMSO) 3.7-3.8(3H, s), 6.8-7.0(3H, m), 7.2-7.3(2H, m), 7.3-7.4(1H, m), 7.7-7.8(1H, s), 8.5-8.6(2H, m), 11.8(0.6H, s) |
| **56** | 347 | 10.38 | (CDCl₃) 2.05-2.14 (4H, m), 3.54-3.61 (4H, m), 6.65 (1H, s), 7.36-7.43 (1H, m), 7.46-7.54 (2H, m), 7.76-7.83 (2H, m), 7.86 (1H, s), 8.54-8.59 (2H, m), 9.56 (1H, br s) |
| **57** | 379 | 10.39 | (CDCl₃) 1.27-1.33 (3H, m), 3.43 (3H, s), 3.55-3.62 (2H, m), 3.67-3.77 (4H, m), 6.64 (1H, s), 7.35-7.42 (1H, m), 7.46-7.54 (2H, m), 7.66-7.71 (2H, m), 7.80 (1H, s), 8.60 (2H, s), 9.42 (1H, br s) |
| **58** | 335 | 10.35 | (CDCl₃) 1.24-1.34 (3H, m), 3.27 (3H, s), 3.55-3.64 (2H, m), 6.64 (1H, s), 7.35-7.41 (1H, m), 7.45-7.53 (2H, m), 7.65-7.72 (2H, m), 7.85 (1H, s), 8.58-8.63 (2H, m), 9.57 (1H, br s) |
| **59** | 376 | 9.82 | (CDCl₃) 2.41(3H, s), 2.53-2.63 (4H, m), 3.58-3.66 (4H, m), 6.72 (1H, s), 7.37-7.43 (1H, m), 7.46-7.53 (2H, m), 7.65-7.71 (2H,m), 8.52 (1H, s), 8.60 (1H, s), 9.39 (1H, br s) |
| **60** | 365 | 9.61 | (CDCl₃) 1.29-1.35 (3H, m), 3.46-3.53 (2H, m), 3.74-3.81 (2H, m), 3.95-4.02 (2H, m), 6.66 (1H, s), 7.34-7.40 (1H, m), 7.47-7.54 (2H,m), 7.65-7.70 (2H, m), 7.78 (1H, s), 8.44 (1 H, s), 8.61 (1H, s), 9.80 (1H, br s) |
| **61** | 363 | 9.61 | (DMSO) 1.14 (6H, d, J = 6.8Hz), 2.72-2.84 91H, m), 7.35-7.56 (4H, m), 7.75-7.82 (2H, m), 7.90 (1H, brs), 8.58 (1H, brs), 8.70 (1H, brs), 11.98 (1H, brs), 12.13 (1H, brs). |
| **62** | 363 | 10.97 | (CDCl₃) 0.80-0.92 (6H, m), 1.25-1.35 (3H, m), 3.41-3.52 (2H, m), 4.25-4.35 (1H, m), 6.60 (1H, s), 7.33-7.40 (1H, m), 7.45-7.52 (2H, m), 7.64-7.71 (2H, m), 7.81 (1H, s), 7.56 (1H, s), 7.60 (1H, s), 9.90 (1H, br s) |
| **63** | 392 | 10.27 | (CDCl₃) 1.34 (3H, t), 2.46 (6H, s), 2.82-2.99 (2H, m), 3.52-3.59 (2H, q), 3.65-3.80 (2H, m), 6.66 (1H, s), 7.35-7.41 (1H, m), 7.43-7.52 (2H, m), 7.63-7.70 (2H, m), 7.80 (1H, s), 8.56 (1H, s), 8.60 (1H, s), 9.29 (1H, br s) |
| **64** | 381 | 9.64 | (DMSO) 1.14 (6H, d, J = 6.9Hz), 2.72-2.86 (1H, m), 7.30-7.40 (2H, m), 7.52 (1H, s), 7.79-7.91 (3H, m), 8.56 (1H, brs), 8.66 (1H, brs), 11.99 (1H, brs), 12.12 (1H, brs). |
| **65** | 353 | 8.98 | (DMSO) 1.15 (6H, d, J - 6.8Hz), 2.72-2.86 (1H, m), 7.18 (1H, s), 7.53 (1H, s), 7.78-7.89 (2H, m), 8.30 (1H, s), 8.56-8.62 (1H, m), 11.90 (1H, brs), 12.14 (1H, brs). |
| **66** | 298 | 9.58 | (DMSO) 1.21 (6H, t, J = 7.0Hz), 3.51 (4H, q, J = 7.0Hz), 7.08 (1H, s), 8.04 (1H, brs), 8.65 (1H, brs), 8.91 (1H, brs), 12.48 (1H, brs). |
| **67** | 405 | 10.68 | (DMSO) 1.00 (6H, d), 1.40 (3H, t), 2.11-2.23 (1H, m), 2.63 (2H, d), 4.34 (2H, q), 7.34-7.41 (1H, m), 7.46-7.54 (3H, m), 7.73-7.75 (2H, m), 7.99 (1H, s), 8.56 (1H, s), 8.76 (1H, s), 11.95 (1H, s) |
| **68** | 363 | 9.80 | (DMSO) 1.42 (3H, t), 2.46 (3H, s), 4.35 (2H, q), 7.34-7.40 (1H, m), 7.51-7.56 (3H, m), 7.76-7.71 (2H, m), 8.01 (1H, s), 8.60 (1H, s), 8.74 (1H, s), 12.00 (1H, s) |
| **69** | 349 | 10.59 | (DMSO) 1.2 (6H, q), 3.5 (4H, t), 6.9 (1H, s), 7.4 (1H, m), 7.5 (2H, m), 7.7 (1H, m), 7.9 (1H, s), 8.1 (2H, m), 8.5 (1H, d), 11.8 (NH, s) |
| **70** | 392 | 9.9 | (DMSO) 1.2-1.3(6H, m), 3.5-3.6(4H, m), 6.9(1H, s), 7.5-7.6(3H, m), 7.7(1H, s), 7.9-8.1 (3H, m), 8.4-8.5(1H, d), 10.6(1H, s), 11.5-11.6(1H, s) |
| **71** | 364 | 10.1 | (DMSO) 1.2-1.3(6H, m), 3.5(4H, m), 6.6-6.7(1H, d), 6.8(1H, s), 6.8-6.9(1H, m), 7.2-7.3(2H, m), 7.4(1H, s), 7.8(2H, m), 8.2(1H, d), 9.0(1H, s), 11.3-11.4(1H, s) |
| **72** | 311 | 8.82 | (CDCl3) 1.51 (3H, t), 2.48 (3H, s), 3.18 (1H, s), 4.42 (2H, q), 7.10 (1H, s), 7.84 (1H, s), 8.51 (1H, s), 8.66 (1H, s), 9.91 (1H, br s) |
| **73** | 406 | 7.73 | (DMSO) 1.14 (6H, d, J =6.8HZ), 2.74-2.86 (1H, m), 7.42 (1H, brs), 7.58 (1H, s), 7.84-7.94 (3H, m), 7.98-8.12 (3H, m), 8.65 (1H, brs), 8.75 (1H, brs), 12.01 (1H, brs), 12.15 (1H, brs). |
| **74** | 330 | 8.32 | (DMSO) 1,23 (6H, t, J = 7.0Hz), 2.85 (3H, d, J = 4.4Hz), 3.52 (4H, q, J = 7.0Hz), 6.98 (1H, s), 7.90 (1H, brs), 8.50-8.60 (1H, m), 8.74 (1H, brs), 8.86 (1H, brs), 12.01 (1H, brs). |
| **75** | 297 | 9.86 | (DMSO) 1.31 (6H, t, J = 7.0Hz), 3.16 (1H, s), 3.59 (4H, q, J = 7.0Hz), 6.60 (1H, s), 7.80 (1H, brs), 8.49 (1H, brs), 8.55 (1H, brs), 9.75 (1H, brs). |

**Example 4**

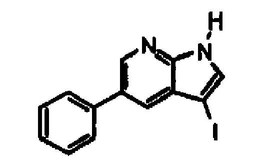

**3-iodo-5-phenyl-1H-pyrrolo[2,3-b]pyridine**

A solution of 5-phenyl-1H-pyrrolo[2,3-b]pyridine (2.96g, 15.24mmol, 1eq) in anhydrous DMF (60ml), stirring at ambient temperature, was treated with iodine (7.74g, 30.50mmol, 2eq) and then potassium hydroxide (3.20g, 57.14mmol, 3.75eq). The reaction mixture was stirred at room temperature for 15h before being diluted with a mixture of aqueous sodium thiosulfate and ethyl acetate. The organic layer was separated, washed with a saturated aqueous sodium chloride solution, dried with sodium sulfate, filtered and then concentrated in-vacuo. The resulting oil was then dissolved in a DCM/MeOH mixture and adsorbed onto silica gel. The material was then dry-loaded onto a column and subjected to silica-gel chromatography using a mixture of ethyl acetate (1): 40-60 petroleum ether (2) as eluent to yield 3-iodo-5-phenyl-1H-pyrrolo[2,3-b]pyridine (1) (3.16g, 65%) as a white solid.1H NMR, (400Mhz, DMSO) 7.34-7.41 (1H, m), 7.46-7.56 (2H, m), 7.70-7.80 (3H, m), 7.81-7.89 (1H, m), 8.53-8.60 (1H, m), 12.21 (1H, brs).
**Example 5**

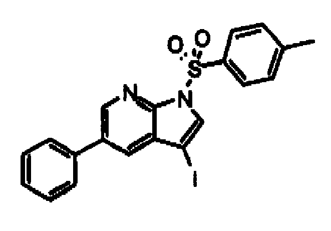

**3-iodo-5-phenyl-1-tosyl-1H-pyrrolo[2,3-b]pyridine**

A suspension of 60% sodium hydride in mineral oil (79mg, 1.98mmol, 1.2eq) in anhydrous DMF (30ml), stirring at ambient temperature, was treated with a solution of 3-iodo-5-phenyl-1H-pyrrolo[2,3-b]pyridine (1) (530mg, 1.66mmol, 1.0eq) in DMF (5ml). The reaction mixture was then stirred at room temperature for 1h before being cooled to 0oC. A solution of p-toluenesulfonyl chloride (316mg, 1.66mmol, 1.0eq) in anhydrous DMF (5ml) was then added and the reaction mixture allowed to warm to room temperature over 15h. The reaction mixture was then diluted with a mixture of water and ethyl acetate, washed with a saturated aqueous sodium chloride solution, dried with sodium sulfate, filtered and then concentrated in-vacuo. The resulting oil was then subjected to silica-gel chromatography using a mixture of ethyl acetate (1): 40-60 petroleum ether (2) as eluent to yield 3-iodo-5-phenyl-1-tosyl-1H-pyrrolo[2,3-b]pyridine (2) (743mg, 95%) as a white solid. 1H NMR, (400Mhz, DMSO) 2.37 (3H, s), 7.39-7.56 (5H, m), 7.75 (2H, d), 7.91 (1H, s), 8.05 (2H, d), 8.20 (1H, s), 8.71 (1H, s).
**Example 6** 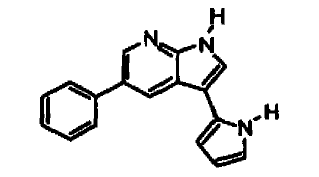 **5-phenyl-3-(1H-pyrrol-2-yL)-1-H pyrrolo[2,3-b]pyridine**

A mixture of 3-iodo-5-phenyl-1-tosyl-1H-pyrrolo[2,3-b]pyridine (2) (150mg, 0.32mmol, 1eq), tetrakis(triphenylphosphine)palldium(0) (4mg, 0.0035mmol, 0.01eq) and 1-(tert-butoxycarbonyl)-1H-pyrrol-2-yl-2-boronic acid (67mg, 0.32mmol, 1eq) was placed into a microwave tube. The mixture was then treated with DME (4ml), EtOH (0.86ml), water (1.14ml) and an aqueous 2N sodium carbonate solution (0.63ml). The tube was placed into the microwave and heated at 160oC for 40min. The tube was allowed to cool to room temperature and diluted with water/ethyl acetate. The organic layer was separated, dried over sodium sulfate and concentrated in-vacuo to yield a gum. The gum was dissolved in DMSO and subjected to reverse-phase chromatography using ACN/water as a gradient eluent to yield 5-phenyl-3-(1H-pyrrol-2-yl)-1-H-pyrrolo[2,3-b]pyridine (3) as a solid. 1H NMR, (400Mhz, DMSO) 6.10-6.16 (1H, m), 6.45-6.50 (1H, m), 6.78-6.84 (1H, m), 7.32-7.54 (3H, m), 8.37-8.42 (1H, m), 8.52-8,58 (1H, m), 11.05 (1H, brs), 11.75 (1H, brs).

A variety of other compounds of Formula I have been prepared by methods substantially similar to those described herein Example 6. The characterization data for these compounds is summarized in Table 4 below and includes HPLC, LC/MS (observed) and ¹H NMR data.

**Table 4. Characterization Data for Selected Compounds of Formula I**

| **Compound No II-** | **M+1(obs)** | **Rt (min)** | **¹H-NMR** |
|---|---|---|---|
| **2** | 319 | 9.70 | (DMSO) 2.55 (3H, s), 7.36-7.45 (1H, m), 7.47-7.59 (2H, m), 7.61-7.69 (1H, m), 7.75-7.85 (2H, m), 7.92-7.99 (1H, m), 8.19-8.25 (1H, m), 8.41-8.48 91H, m), 8.59-8.66 91H, m), 12.34 (1H, brs). |

### Example 7: ITK Inhibition Assay:

Compounds were screened for their ability to inhibit Itk using a radioactive-phosphate incorporation assay. Assays were carried out in buffer consisting of 100 mM HEPES (pH 7.4), 10mM MgCl₂, 25mM NaCl, 0.01% BSA and 1mM DTT at 25 deg C. in the presence of 30 nM Itk. Final substrate concentrations were 15 µM [γ-³³P]ATP (400µCi ³³P ATP/ µmol ATP, Amersham Pharmacia Biotech / Sigma Chemicals) and 2µM peptide (SAM68 Δ332-443). An assay stock buffer solution was prepared containing all of the reagents listed above, with the exception of ATP and the test compound of interest. 50 µL of the stock solution was placed in a 96 well plate followed by addition of 1.5µL of DMSO stock containing serial dilutions of the test compound (typically starting from a final concentration of 15µM with 2-fold serial dilutions) in duplicate (final DMSO concentration 1.5%). The plate was pre-incubated for 10 minutes at 25°C and the reaction initiated by addition of 50µL [γ-³³P]ATP (final concentration 15µM).

The reaction was stopped after 10 minutes by the addition of 50µL of a TCA / ATP mixture (20% TCA, 0.4mM ATP). A Unifilter GF/C 96 well plate (Perkin Elmer Life Sciences, Cat no. 6005174) was pretreated with 50µL Milli Q water prior to the addition of the entire reaction mixture (150 µL). The plate was washed with 200µL Milli Q water followed by 200µL of a TCA / ATP mixture (5% TCA, 1mM ATP). This wash cycle was repeated a further 2 times. After drying, 30µL Optiphase 'Supermix' liquid scintillation cocktail (Perkin Elmer) was added to the well prior to scintillation counting (1450 Microbeta Liquid Scintillation Counter, Wallac).

IC50 data were calculated from non-linear regression analysis of the initial rate data using the Prism software package (GraphPad Prism version 3.0a for Macintosh, GraphPad Software, San Diego California, USA).

Assays were carried out in a mixture of 20 mM MOPS (pH 7.0), 10mM MgCl2, 0.1% BSA and 1mM DTT. Final substrate concentrations in the assay were 7.5 µM [γ-33P]ATP (400mCi 33P ATP/ mmol ATP, Amersham Pharmacia Biotech / Sigma Chemicals) and 3µM peptide (SAM68 protein D332-443). Assays were carried out at 25 °C. in the presence of 50 nM Itk. An assay stock buffer solution was prepared containing all of the reagents listed above, with the exception of ATP and the test compound of interest. 50 µL of the stock solution was placed in a 96 well plate followed by addition of 2µL of DMSO stock containing serial dilutions of the test compound (typically starting from a final concentration of 50µM with 2-fold serial dilutions) in duplicate (final DMSO concentration 2%). The plate was pre-incubated for 10 minutes at 25°C and the reaction initiated by addition of 50µL [γ-33P]ATP (final concentration 7.5µM).

The reaction was stopped after 10 minutes by the addition of 100mL 0.2M phosphoric acid + 0.01% TWEEN 20. A multiscreen phosphocellulose filter 96-well plate (Millipore, Cat no. MAPHN0B50) was pretreated with 100µL 0.2M phosphoric acid + 0.01% TWEEN 20 prior to the addition of 170mL of the stopped assay mixture. The plate was washed with 4 x 200µL 0.2M phosphoric acid + 0.01% TWEEN 20. After drying, 30µL Optiphase 'SuperMix' liquid scintillation cocktail (Perkin Elmer) was added to the well prior to scintillation counting (1450 Microbeta Liquid Scintillation Counter, Wallac).

Ki(app) data were calculated from non-linear regression analysis of the initial rate data using the Prism software package (GraphPad Prism version 3.0cx for Macintosh, GraphPad Software, San Diego California, USA).

### Example 8: ITK Inhibition Assay (AlphaScreen™):

Compounds were screened for their ability to inhibit Itk using an AlphaScreen™ phosphotyrosine assay at Vertex Pharmaceuticals. Assays were carried out in a mixture of 20 mM MOPS (pH 7.0), 10mM Mgcl₂, 0.1% BSA and 1mM DTT. Final substrate concentrations in the assay were 100µM ATP (Sigma Chemicals) and 2µM peptide (Biotinylated SAM68 Δ332-443). Assays were carried out at 25°C and in the presence of Itk (30nM). An assay stock buffer solution was prepared containing all of the reagents listed above, with the exception of ATP and the test compound of interest. 25 µL of the stock solution was placed in each well of a 96 well plate followed by 1 µL of DMSO containing serial dilutions of the test compound (typically starting from a final concentration of 15µM) in duplicate (final DMSO concentration 2%). The plate was preincubated for 10 minutes at 25°C and the reaction initiated by addition of 25µL ATP (final concentration 100µM). Background counts were determined by the addition of 5µL 500mM EDTA to control wells containing assay stock buffer and DMSO prior to initiation with ATP.

The reaction was stopped after 30 minutes by diluting the reaction 225-fold into MOPS buffer (20mM MOPS (pH 7.0), 1mM DTT, 10mM MgCl₂, 0.1% BSA) containing 50mM EDTA to bring the final concentration of Biotin-SAM68 to 9nM.

Alphascreen™ reagents were prepared according to the manufacturers instructions (AlphaScreen™ phosphotyrosine (P-Tyr-100) assay kit, PerkinElmer catalogue number 6760620C). Under subdued lighting, 20µL of AlphaScreen™ reagents were placed in each well of a white half area 96 well plate (Coming Inc. - COSTAR 3693) with 30µL of the stopped, diluted kinase reactions. Plates were incubated in the dark for 60 minutes prior to reading on a Fusion Alpha plate reader (PerkinElmer).

After removing mean background values for all of the data points, Ki(app) data were calculated from non-linear regression analysis using the Prism software package (GraphPad Prism version 3.0cx for Macintosh, GraphPad Software, San Diego California, USA).

In general, compounds of the invention, including compounds in Table 1 and Table 2, are effective for the inhibition of ITK.

### Example 9: ITK Inhibition Assay (UV):

Compounds were screened for their ability to inhibit Itk using a standard coupled enzyme assay (Fox et al., Protein Sci., (1998) 7, 2249). Assays were carried out in a mixture of 20 mM MOPS (pH 7.0), 10mM MgCl₂, 0.1% BSA, 1mM DTT, 2.5 mM phosphoenolpyruvate, 300 µM NADH, 30 µg/ml pyruvate kinase and 10 µg/ml lactate dehydrogenase. Final substrate concentrations in the assay were 100µM ATP (Sigma Chemicals) and 3µM peptide (Biotinylated SAM68 Δ332-443). Assays were carried out at 25 °C and in the presence of 100nM Itk.

An assay stock buffer solution was prepared containing all of the reagents listed above, with the exception of ATP and the test compound of interest. 60 µl of the stock solution was placed in a 96 well plate followed by addition of 2 µl of DMSO stock containing serial dilutions of the test compound (typically starting from a final concentration of 15 µM). The plate was preincubated for 10 minutes at 25°C and the reaction initiated by addition of 5 µl of ATP. Initial reaction rates were determined with a Molecular Devices SpectraMax Plus plate reader over a 10 minute time course. IC50 and Ki data were calculated from non-linear regression analysis using the Prism software package (GraphPad Prism version 3.0cx for Macintosh, GraphPad Software, San Diego California, USA).

In general, compounds of the invention, including compounds in Table 1 and Table 2, are effective for the inhibition of ITK.

### Example 10: BTK Inhibition Assay:

Compounds were screened for their ability to inhibit Btk using a radioactive-phosphate incorporation assay at Vertex Pharmaceuticals. Assays were carried out in a mixture of 100 mM HEPES (pH 7.5), 10mM MgCl₂, 25mM NaCl, 0.01% BSA and 1mM DTT. Final substrate concentrations in the assay were 100µM ATP (Sigma Chemicals) and 5µM peptide (SAM68 Δ332-443). Assays were carried out at 25°C and in the presence of Btk (25nM) and [γ-³³P]ATP (100µCi ³³P ATP/µmol ATP, Amersham Pharmacia Biotech, Amersham, UK). An assay stock buffer solution was prepared containing all of the reagents listed above, with the exception of SAM68 and the test compound of interest. 75 µL of the stock solution was placed in a 96 well plate followed by addition of 1.5µL of DMSO stock containing serial dilutions of the test compound (typically starting from a final concentration of 15µM) in duplicate (final DMSO concentration 1.5%). The plate was preincubated for 15 minutes at 25°C and the reaction initiated by addition of 25µL SAM68 (final concentration 5µM). Background counts were determined by the addition of 50µL 20% TCA + 0.4mM ATP to control wells containing assay stock buffer and DMSO prior to initiation with SAM68.

The reaction was stopped after 60 minutes by the addition of 50µL 20% TCA + 0.4mM ATP. A Unifilter GF/C 96 well plate (Perkin Elmer Life Sciences, Cat no. 6005174) was pretreated with 50µL Milli Q water prior to the addition of the entire reaction mixture (150 µL). The plate was washed with 200µL Milli Q water followed by 200µL 5% TCA + 1mM ATP. The water/TCA wash cycle was repeated a further 2 times. After drying, 30µL Optiphase 'SuperMix' liquid scintillation cocktail (Perkin Elmer) was added to the well prior to scintillation counting (1450 Microbeta Liquid Scintillation Counter, Wallac).

After removing mean background values for all of the data points, Ki(app) data were calculated from non-linear regression analysis using the Prism software package (GraphPad Prism version 3.0a for Macintosh, GraphPad Software, San Diego California, USA).

Compounds were screened for their ability to inhibit Btk using an AlphaScreen™ phosphotyrosine assay at Vertex Pharmaceuticals. Assays were carried out in a mixture of 20 mM MOPS (pH 7.0), 10mM MgCl₂, 0.1% BSA and 1mM DTT. Final substrate concentrations in the assay were 50µM ATP (Sigma Chemicals) and 2µM peptide (Biotinylated SAM68 Δ332-443). Assays were carried out at 25°C and in the presence of Btk (25nM). An assay stock buffer solution was prepared containing all of the reagents listed above, with the exception of Biotin-SAM68 and the test compound of interest. 37.5µL of the stock solution was placed in each well of a 96 well plate followed by 1µL of DMSO containing serial dilutions of the test compound (typically starting from a final concentration of 15µM) in duplicate (final DMSO concentration 2%). The plate was preincubated for 15 minutes at 25°C and the reaction initiated by addition of 12.5µL Biotin-SAM68 (final concentration 2µM). Background counts were determined by the addition of 5µL 500mM EDTA to control wells containing assay stock buffer and DMSO prior to initiation with Biotin-SAM68.

The reaction was stopped after 30 minutes by diluting the reaction 225-fold into MOPS buffer (20mM MOPS (pH 7.0), 1mM DTT, 10mM MgCl₂, 0.1% BSA) containing 50mM EDTA to bring the final concentration of Biotin-SAM68 to 9nM.

Alphascreen™ reagents were prepared according to the manufacturers instructions (AlphaScreen™ phosphotyrosine (P-Tyr-100) assay kit, PerkinElmer catalogue number 6760620C). Under subdued lighting, 20µL of AlphaScreen™ reagents were placed in each well of a white half area 96 well plate (Coming Inc. - COSTAR 3693) with 30µL of the stopped, diluted kinase reactions. Plates were incubated in the dark for 60 minutes prior to reading on a Fusion Alpha plate reader (PerkinElmer).

After removing mean background values for all of the data points, Ki(app) data were calculated from non-linear regression analysis using the Prism software package (GraphPad Prism version 3.0cx for Macintosh, GraphPad Software, San Diego California, USA).

In general, compounds of the invention, including compounds in Table 1 and Table 2, are effective for the inhibition of Btk.

### Example 11: RLK Inhibition Assay:

Compounds were screened for their ability to inhibit Rlk using a standard coupled enzyme assay (Fox et al., Protein Sci., (1998) 7, 2249). Assays were carried out in a mixture of 20 mM MOPS (pH 7.0), 10mM MgCl2, 0.1% BSA and 1mM DTT. Final substrate concentrations in the assay were 100µM ATP (Sigma Chemicals) and 10µM peptide (Poly Glu:Tyr 4:1). Assays were carried out at 30 °C and in the presence of 40nM Rlk. Final concentrations of the components of the coupled enzyme system were 2.5 mM phosphoenolpyruvate, 300 µM NADH, 30 µg/ml pyruvate kinase and 10 µg/ml lactate dehydrogenase.

An assay stock buffer solution was prepared containing all of the reagents listed above, with the exception of ATP and the test compound of interest. 60 µl of the stock solution was placed in a 96 well plate followed by addition of 2 µl of DMSO stock containing serial dilutions of the test compound (typically starting from a final concentration of 7.5µM). The plate was preincubated for 10 minutes at 30°C and the reaction initiated by addition of 5 µl of ATP. Initial reaction rates were determined with a Molecular Devices SpectraMax Plus plate reader over a 10 minute time course. IC50 and Ki data were calculated from non-linear regression analysis using the Prism software package (GraphPad Prism version 3.0cx for Macintosh, GraphPad Software, San Diego California, USA).

In general, compounds of the invention, including compounds in Table 1 and Table 2, are effective for the inhibition of RLK.

### Example 12; JAK3 Inhibition Assay:

Compounds were screened for their ability to inhibit JAK using the assay shown below. Reactions were carried out in a kinase buffer containing 100 mM HEPES (pH 7.4), 1 mM DTT, 10 mM MgCl₂, 25 mM NaCl, and 0.01 % BSA.

Substrate concentrations in the assay were 5 µM ATP (200 uCi/µmole ATP) and 1 µM poly(Glu)₄Tyr. Reactions were carried out at 25 °C and 1 nM JAK3.

To each well of a 96 well polycarbonate plate was added 1.5 µl of a candidate JAK3 inhibitor along with 50 µl of kinase buffer containing 2 µM poly(Glu)₄Tyr and 10 µM ATP. This was then mixed and 50µl of kinase buffer containing 2 nM JAK3 enzyme was added to start the reaction. After 20 minutes at room temperature (25C), the reaction was stopped with 50µl of 20% trichloroacetic acid (TCA) that also contained 0.4 mM ATP. The entire contents of each well were then transferred to a 96 well glass fiber filter plate using a TomTek Cell Harvester. After washing, 60 µl of scintillation fluid was added and ³³P incorporation detected on a Perkin Elmer TopCount.

In general, compounds of the invention, including compounds in Table 1 and Table 2, are effective for the inhibition of JAK (e.g., JAK-3).

The present invention further comprises the aspects defined in the following clauses (which form part of the present description but are not considered as claims in accordance with decision J 15/88 of Legal Board of Appeal of the European Patent Office):
1. A compound of formula (I): 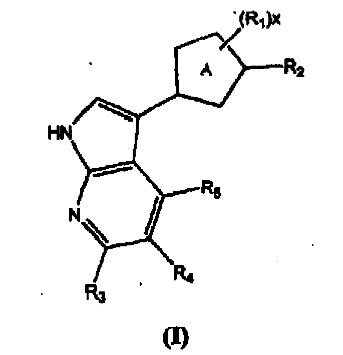 or a pharmaceutically acceptable salt thereof, wherein:
   Ring A is an optionally substituted five membered ring selected from: 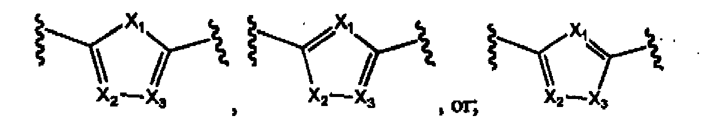 x is 0,1 or 2;
      each occurrence of R¹ is halogen, CN, NO₂, or UₘR;
      R² is independently selected from Tₙ-R'
      X¹, X² and X³ are each independently CR¹, N, S or O;
      R³, R⁴, and R⁵ are each independently halogen, CN, NO₂, or V_{P}-R';
      each occurrence of T, U or V is independently an optionally substituted C₁₋₆ alkylidene chain, wherein up to two methylene units of the chain are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-;
   m , n and p are each independently 0 or 1; each occurrence of R is independently hydrogen or an optionally substituted C₁₋₆ aliphatic group; and each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R', two occurrences of R, or two occurrences of R', are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur; provided that
      at least one occurrence of R³, R⁴, and R⁵ is Vₚ-R', wherein R' is not hydrogen;
      if n is 0, then R' is not H;
      if Ring A is 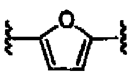 and R⁴ is 2-phenoxylphenyl, then R² is not COOH or CONHR^{x} wherein R^{x} is *n*-propyl, phenyl, cyclohexyl, benzyl, -CH₂CH₂OH, -CH₂-cycloproyl, -CH₂CH₂OCH₃, 3-pyridyl, 4-hydroxy-cyclohexyl, or -CH₂-C≡CH.
2. The compound of clause 1, wherein one of R³, R⁴, and R⁵ is Vₚ-R', wherein, R' is an optionally substituted 5- or 6-membered fully unsaturated monocyclic ring having 0-3 heteroatoms independently selected from nitrogen, oxygen, or sulfur, or an optionally substituted 9- or 10-membered fully unsaturated bicyclic ring system having 0-5 heteroatoms independently selected from nitrogen, oxygen, or sulfur.
3. The compound of clause 1, wherein one of R³, R⁴, and R⁵ is Vₚ-R', wherein R' is an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur.
4. The compound of any one of clauses 1-3, wherein R⁴ is Vₚ-R', wherein R' is an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur.
5. The compound of clause 4, wherein R⁴ is Vₚ-R', wherein R' is an optionally substituted C₁₋₆ aliphatic group or an optionally substituted 3-8-membered saturated, partially unsaturated, or fully unsaturated monocyclic ring having 0-3 heteroatoms independently selected form nitrogen, oxygen, or sulfur.
6. The compound of clause 5, wherein R⁴ is Vₚ-R', and R' is C≡CH.
7. The compound of clause 5, wherein R⁴ is Vₚ-R', and R is an optionally substituted a 3-8-membered saturated, partially unsaturated, or fully unsaturated monocyclic ring having 0-3 heteroatoms independently selected from nitrogen, oxygen, or sulfur.
8. The compound of clause 7, wherein R⁴ is Vₚ-R', wherein R' is an optionally substituted a 5-6-membered fully unsaturated monocyclic ring having 0-3 heteroatoms independently selected from nitrogen, oxygen, or sulfur.
9. The compound of clause 8, wherein R⁴ is Vₚ-R', and R is an optionally substituted a 6-membered fully unsaturated monocyclic ring having 0-3 nitrogen heteroatoms.
10. The compound of clause 9, wherein the 6-membered fully unsaturated monocyclic ring has 0-1 nitrogen heteroatoms.
11. The compound of clause 7, wherein R⁴ is Vₚ-R', and R' is an optionally substituted a 3-8-membered saturated monocyclic ring having 0-3 heteroatoms independently selected from nitrogen, oxygen, or sulfur.
12. The compound of clause 11, wherein R⁴ is Vₚ-R', and R' is an optionally substituted a 6-membered saturated monocyclic ring having 0-2 heteroatoms independently selected from nitrogen, oxygen, or sulfur.
13. The compound of any one of clauses 1-12, wherein p is 0.
14. The compound of any one of clauses 1-12, wherein p is 1.
15. The compound of clause 14, wherein V is -NR-, -S-, or -O-.
16. The compound of any one of clauses 1-15, wherein R³ is Vₚ-R', wherein p is 0 and R' is hydrogen.
17. The compound of any one of clauses 1-16, wherein R⁵ is halogen or Vₚ-R', wherein p is 0 and R' is hydrogen or C₁₋₆ aliphatic.
18. The compound of clause 17, wherein R⁵ is halogen or Vp-R', wherein p is 0 and R' is hydrogen or C₁₋₃ alkyl.
19. The compound of any one of clauses 1-18, wherein Ring A is: 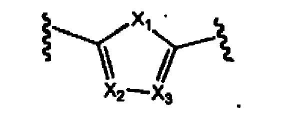
20. The compound of clause 19, wherein X₂ is CR¹.
21. The compound clause 19, wherein ring A is: 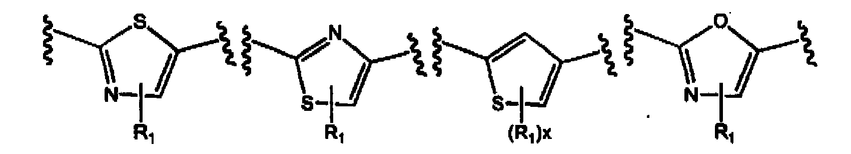 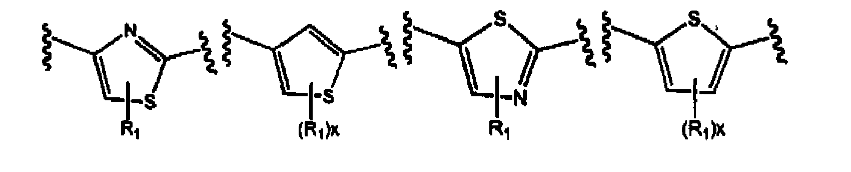 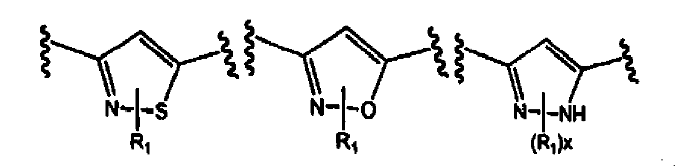 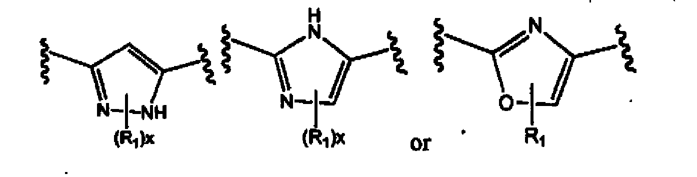
22. The compound of clause 21, wherein ring A is: 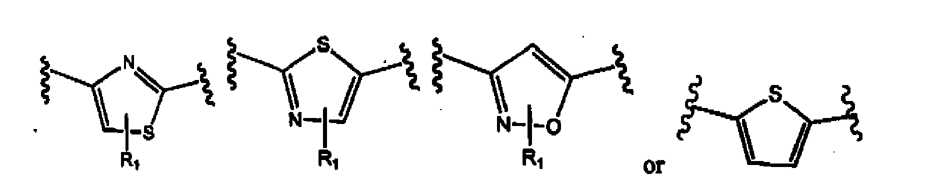
23. The compound of any one of clauses 1-22, wherein R' is UₘR.
24. The compound of any one of clauses 1-23, wherein R² is TₙR', wherein n is 1.
25. The compound of clause 24, wherein T is -NR-, -O-, -CO-, -CONR-, or -NRCO-.
26. The compound of any one of clauses 1-23, wherein R² is TₙR', wherein n is 0.
27. The compound of clause 1, having a formula selected from 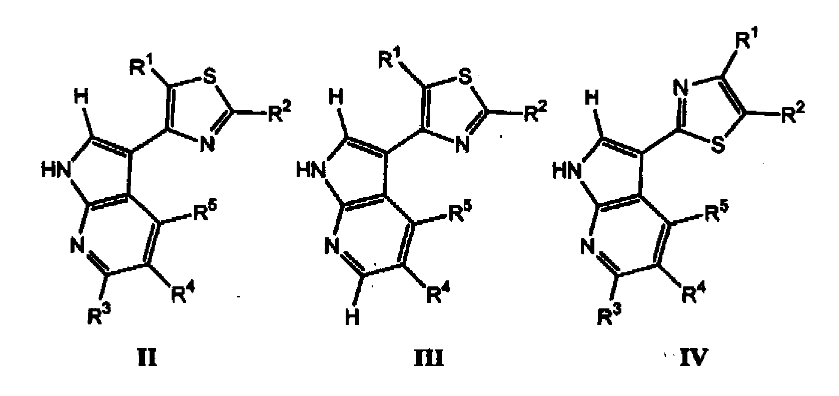 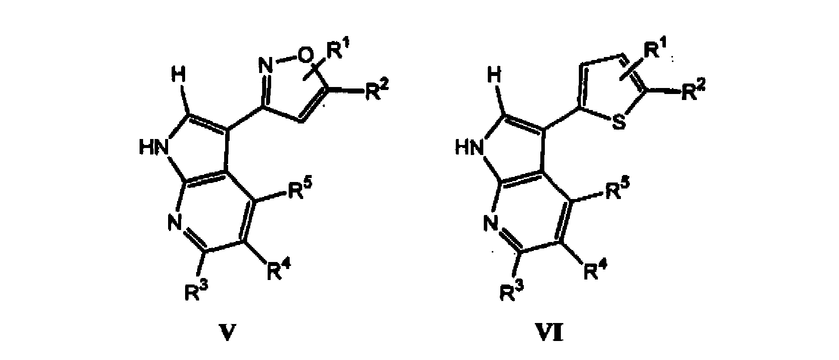 or a phamaceutically acceptable salt thereof.
28. The compound of clause 27, having a formula selected from 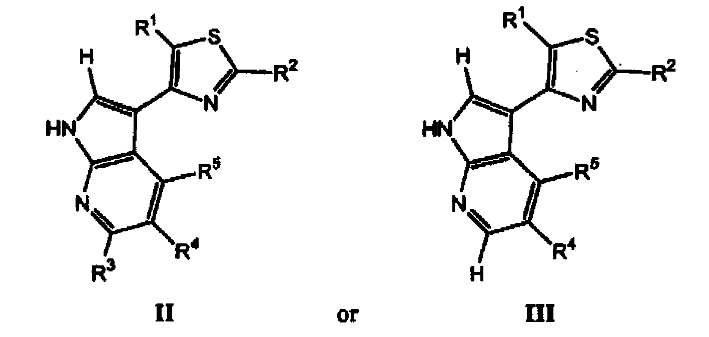 or a pharmaceutically acceptable salt thereof.
29. The compound of any one of clauses 27-28, wherein R¹ is UₘR, wherein m is 0 and R is H or CH₃.
30. The compound of any one of clauses 27-29, wherein R² is TₙR', wherein n is 1.
31. The compound of clause 30, wherein T is -NR-, -O-, -CO-, -CONR-, or -NRCO-.
32. The compound of clause 31, wherein T is -NR-.
33. The compound of clause 32, wherein R and R' are both C₁₋₆aliphatic.
34. The compound of any one of clauses 27-29, wherein R² is TₙR', wherein n is 0.
35. The compound of clause 34, wherein R' is an optionally substituted N-attached heterocyclyl selected from morpholinyl, piperidinyl, pyrrolidinyl, and piperazinyl.
36. The compound of any one of clauses 27-35, wherein R⁴ and R⁵ are each independently Vₚ-R'.
37. The compound of clause 36, wherein R⁴ is Vₚ-R', and R' is C≡CH.
38. The compound of any one of clauses 27-35, wherein one of R³, R⁴, and R⁵ is Vₚ-R', wherein, R' is an optionally substituted 5- or 6-membered fully unsaturated **(i.e., aromatic)** monocyclic ring having 0-3 heteroatoms independently selected from nitrogen, oxygen, or sulfur, or an optionally substituted 9- or 10-membered fully unsaturated bicyclic ring system having 0-5 heteroatoms independently selected from nitrogen, oxygen, or sulfur.
39. The compound of any one of clauses 27-35, wherein one of R³, R⁴, and R⁵ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur.
40. The compound of clause 39, wherein R⁴ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur; and R³ and R⁵ is Vₚ-R', wherein p is 0 and R' is hydrogen.
41. The compound of clause 40, wherein R⁴ is Vₚ-R', wherein R' is independently an optionally substituted C₁₋₆ aliphatic group or an optionally substituted 3-8-membered saturated, partially unsaturated, or fully unsaturated monocyclic ring having 0-3 heteroatoms independently selected from nitrogen, oxygen, or sulfur.
42. The compound of clause 41, wherein R⁴ is Vₚ-R', and R' is an optionally substituted a 3-8-membered saturated, partially unsaturated, or fully unsaturated monocyclic ring having 0-3 heteroatoms independently selected from nitrogen, oxygen, or sulfur.
43. The compound of clause 42, wherein R⁴ is Vₚ-R', wherein R is independently an optionally substituted a 5-6-membered fully unsaturated monocyclic ring having 0-3 heteroatoms independently selected from nitrogen, oxygen, or sulfur.
44. The compound of clause 43, wherein R⁴ is Vₚ-R', and R' is an optionally substituted a 6-membered fully unsaturated monocyclic ring having 0-3 nitrogen heteroatoms.
45. The compound of clause 44, wherein the 6-membered fully unsaturated monocyclic ring has 0-1 nitrogen heteroatoms.
46. The compound of clause 42, wherein R⁴ is Vₚ-R', and R is an optionally substituted a 3-8-membered saturated monocyclic ring having 0-3 heteroatoms independently selected from nitrogen, oxygen, or sulfur.
47. The compound of clause 46, wherein R⁴ is Vₚ-R', and R is an optionally substituted a 6-membered saturated monocyclic ring having 0-2 heteroatoms independently selected from nitrogen, oxygen, or sulfur.
48. The compound of any one of clauses 27-47, wherein p is 1.
49. The compound of clause 48, wherein V is -NR-, -S-, or -O-.
50. The compound of any one of clauses 27-47, wherein p is 0.
51. The compound of any one of clauses 29-50, wherein R³ is Vₚ-R', wherein p is 0 and R' is hydrogen.
52. The compound of any one of clauses 27-51, wherein R⁵ is halogen or Vₚ-R', wherein p is 0 and R' is hydrogen or C₁₋₆ aliphatic.
53. The compound of clause 52, wherein R⁵ is halogen or Vₚ-R', wherein p is 0 and R' is hydrogen or C₁₋₃ alkyl.
54. The compound of clause 1, selected from: 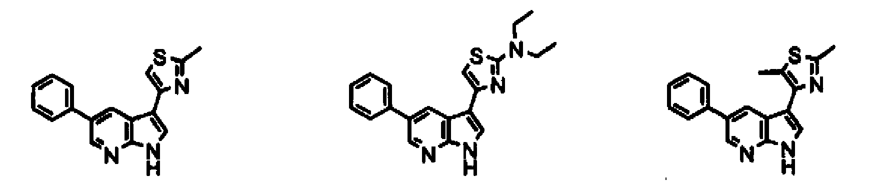 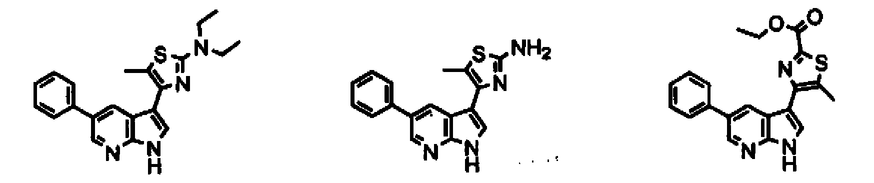 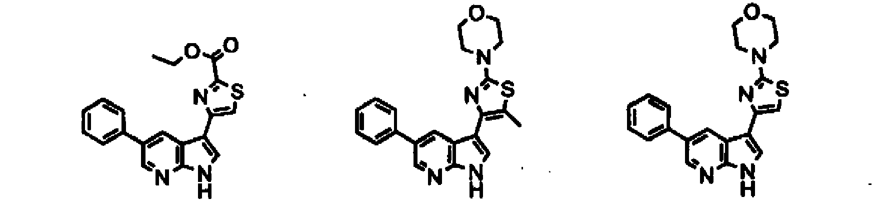 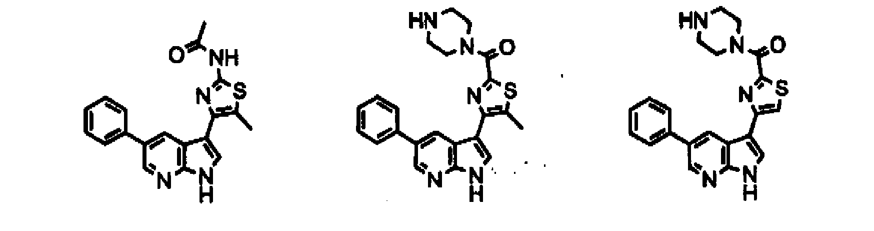 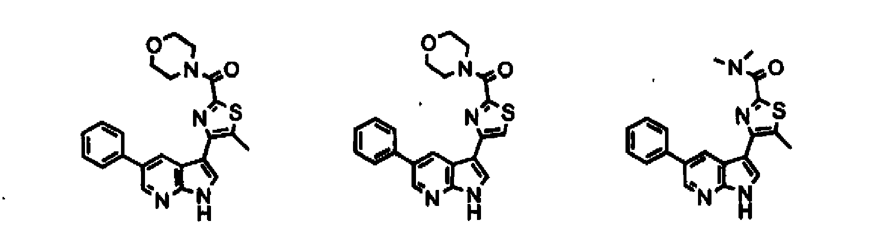 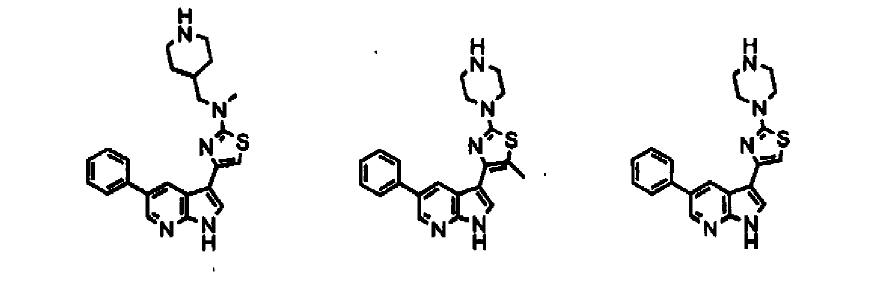 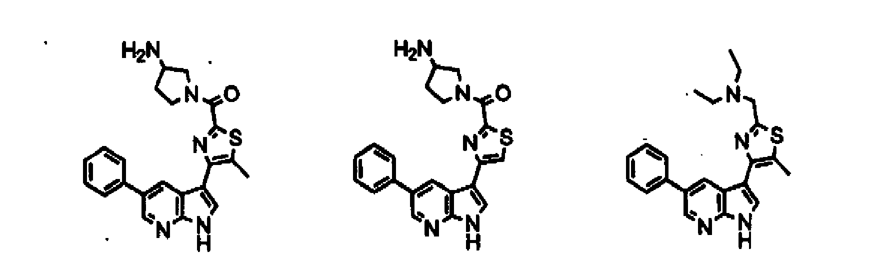 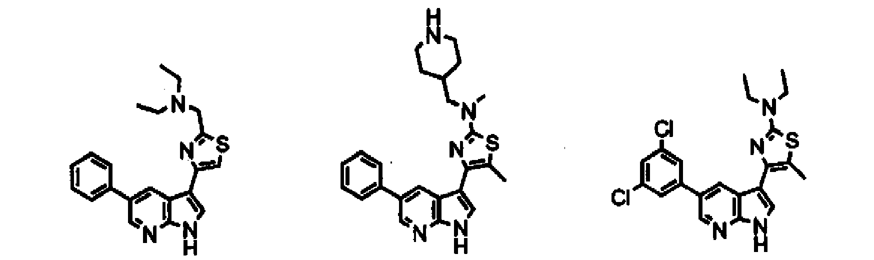 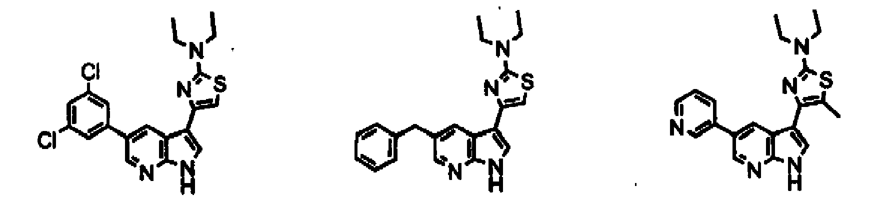 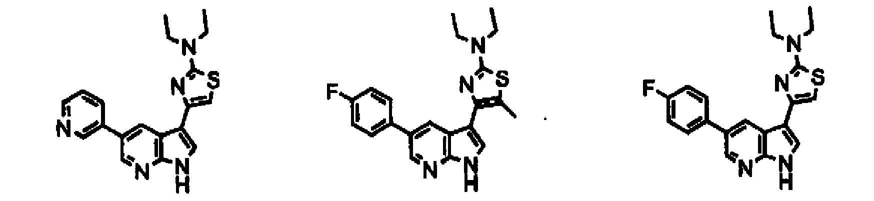 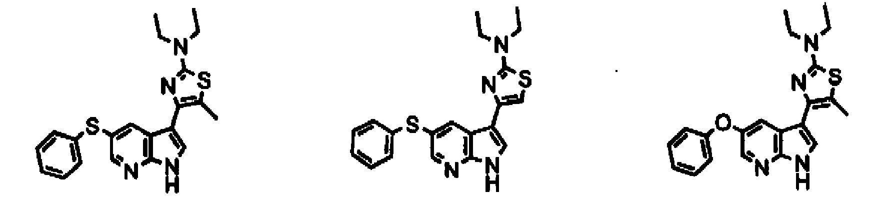 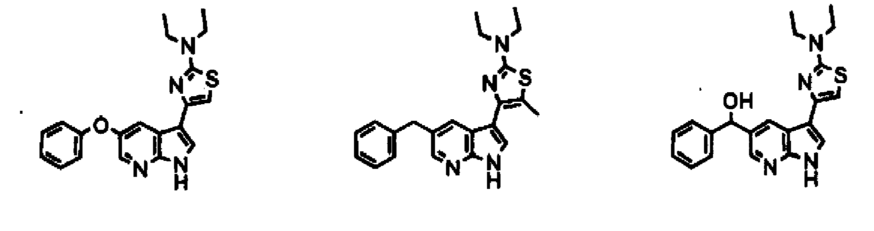 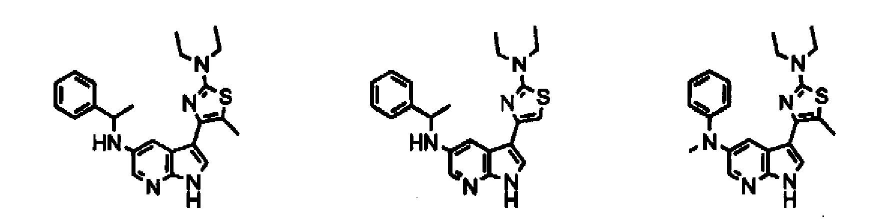 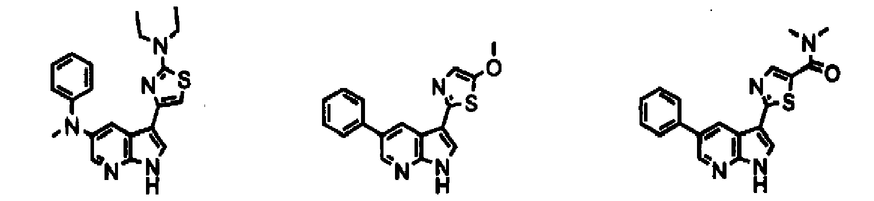 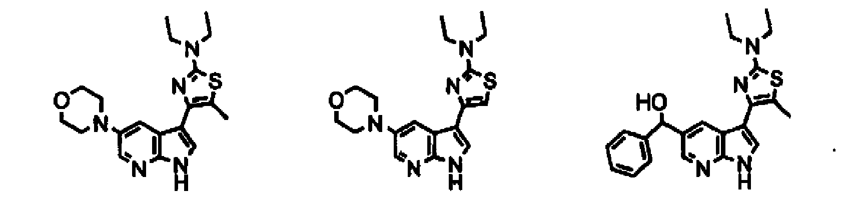 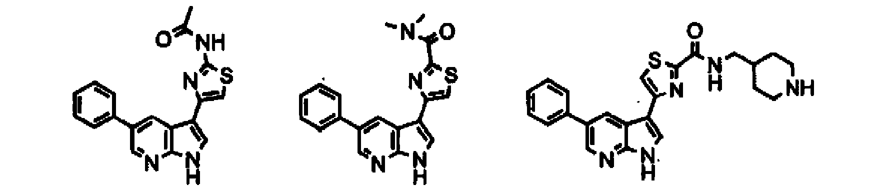 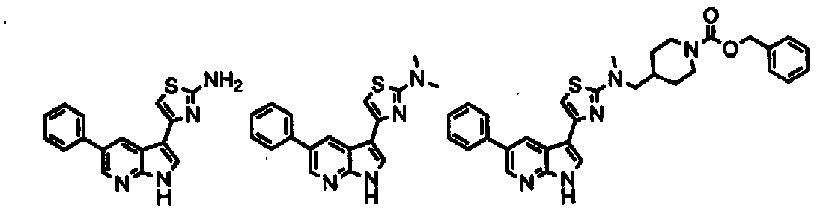 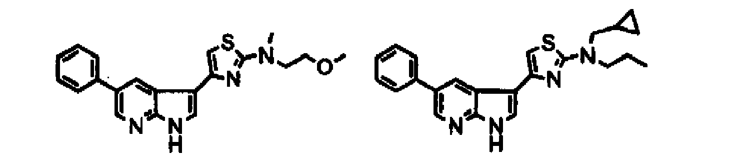 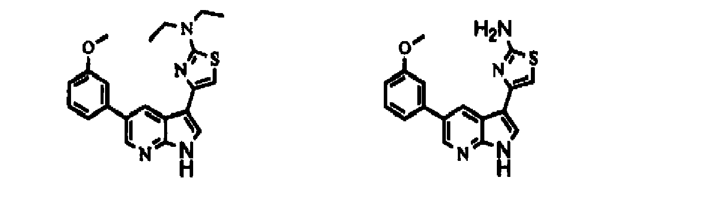 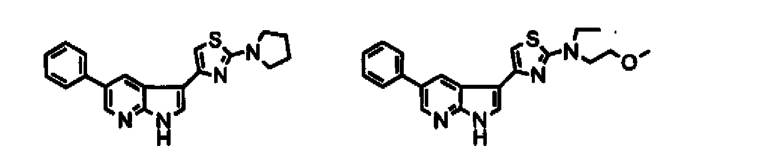 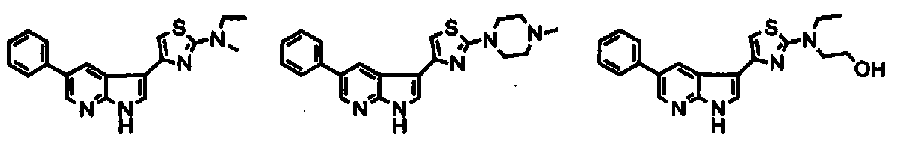 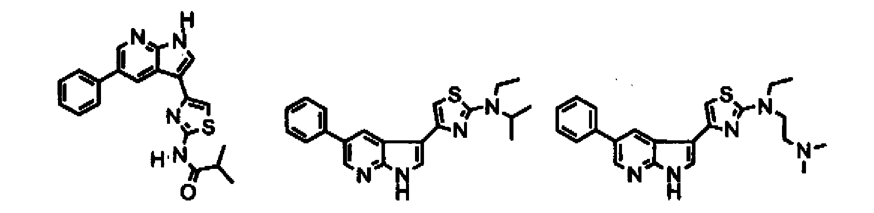 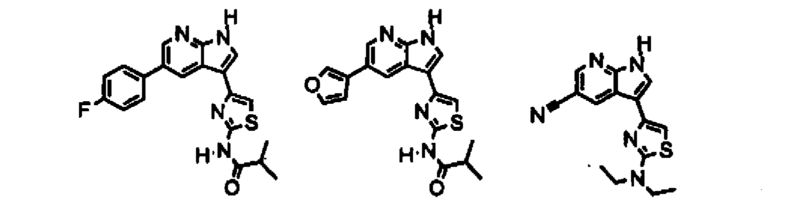 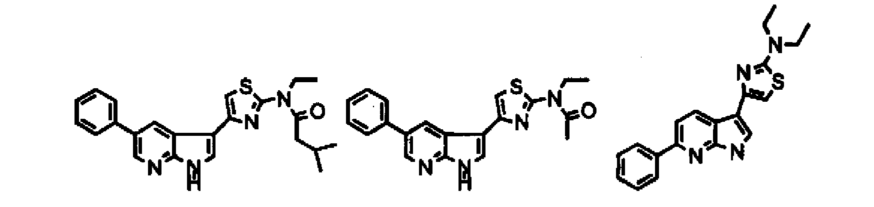 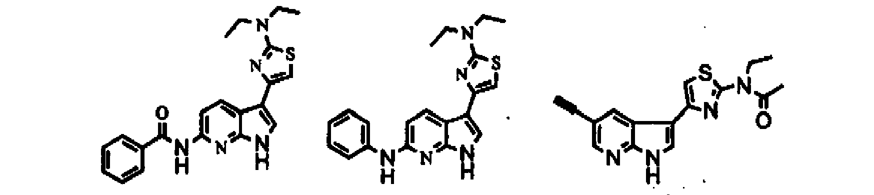 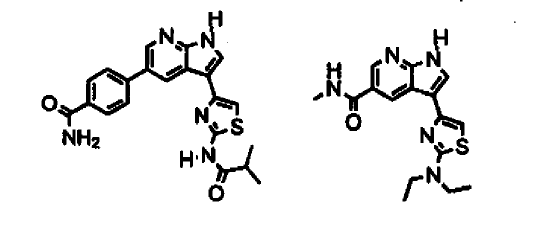 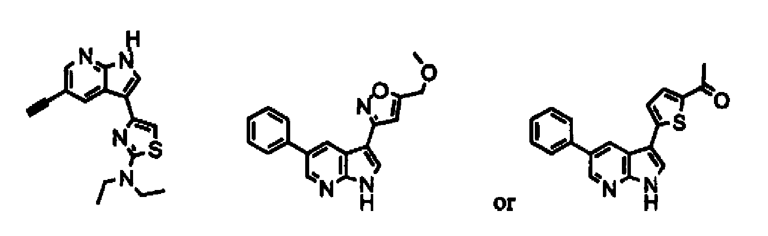
55. A composition comprising a compound of any one of clauses 1-54 or a pharmaceutically acceptable salt thereof, and a pharmaceutically acceptable carrier or diluent.
56. The composition of clause 55, further comprising an additional therapeutic agent selected from an agent for the treatment of an autoimmune, inflammatory, proliferative, hyperproliferative disease, or an immunologically-mediated disease including rejection of transplanted organs or tissues and Acquired Immunodeficiency Syndrome (AIDS).
57. A method of inhibiting Tec family (e.g., Tec, Btk, Itk/Emt/Tsk, Bmx, Txk/Rlk) kinase activity in:
   (a) a patient; or
   (b) a biological sample;
      which method comprises administering to said patient, or contacting said biological sample with a compound according to any one of clauses 1-54.
58. A method of treating or lessening the severity of a disease of condition selected from an autoimmune, inflammatory, proliferative, or hyperproliferative disease or an immunologically-mediated disease comprising administering to a patient in need thereof a composition comprising a compound according to any one of clauses 1-54. clause
59. The method of clause 58, comprising the further step of administering to said patient an additional therapeutic agent selected from an agent for the treatment of an autoimmune, inflammatory, proliferative, hyperproliferative disease, or an immunologically-mediated disease including rejection of transplanted organs or tissues and Acquired Immunodeficiency Syndrome (AIDS), wherein:
   said additional therapeutic agent is appropriate for the disease being treated; and
   said additional therapeutic agent is administered together with said composition as a single dosage form or separately from said composition as part of a multiple dosage form.
60. The method of clause 58 or clause 59, wherein the disease or disorder is asthma, acute rhinitis, allergic, atrophic rhinitis, chronic rhinitis, membranous rhinitis, seasonal rhinitis, sarcoidosis, farmer's lung, fibroid lung, idiopathic interstitial pneumonia, rheumatoid arthritis, seronegative spondyloarthropathis (including ankylosing spondylitis, psoriatic arthritis and Reiter's disease), Behcet's disease, Sjogren's syndrome, systemic sclerosis, psoriasis, systemic sclerosis, atopical dermatitis, contact dermatitis and other eczematous dermatitis, seborrhoetic dermatitis, Lichen planus, Pemphigus, bullous Pemphigus, epidermolysis bullosa, urticaria, angiodermas, vasculitides, erythemas, cutaneous eosinophilias, uveitis, Alopecia, areata vernal conjunctivitis, Coeliac disease, proctitis, eosinophilic gastro-enteritis, mastocytosis, pancreatitis, Crohn's disease, ulcerative colitis, food-related allergies, multiple sclerosis, artherosclerosis, acquired immunodeficiency syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia purpura, restenosis following angioplasty, tumours, artherosclerosis, systemic lupus erythematosus, allograft rejection including without limitation, acute and chronic allograft rejection following for example transplantation of kidney, heart, liver, lung, bone marrow, skin and cornea, and chronic graft versus host disease.

## Claims

1. A compound of formula (I): 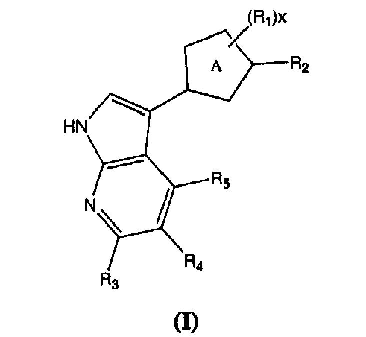 or a pharmaceutically acceptable salt thereof, wherein:
Ring A is an optionally substituted five membered ring selected from: 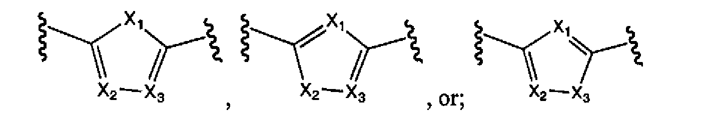 x is 0, 1 or 2;
each occurrence of R¹ is halogen, CN, NO₂ or UₘR;
R² is independently selected from Tₙ-R'
X¹, X² and X³ are each independently CR¹, N, S or O;
R³, R⁴, and R⁵ are each independently halogen, CN, NO₂, or Vₚ-R';
each occurrence of T, U or V is independently an optionally substituted C₁₋₆ alkylidene chain, wherein up to two methylene units of the chain are optionally and independently replaced by -NR-, -S-, -O-, -CS-, -CO₂- -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, or -SO₂-;
m , n and p are each independently 0 or 1; each occurrence of R is independently hydrogen or an optionally substituted C₁₋₆ aliphatic group; and each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R , two occurrences of R, or two occurrences of R', are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur; provided that
at least one occurrence of R³, R⁴, and R⁵ is Vₚ-R', wherein R' is not hydrogen;
if n is 0, then R' is not H;
if Ring A is 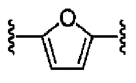 and R⁴ is 2-phenoxylphenyl, then R² is not COOH or CONHR^{x} wherein R^{x} is n-propyl, phenyl, cyclohexyl, benzyl, -CH₂CH₂OH, -CH₂-cycloproyl, -CH₂CH₂OCH₃, 3-pyridyl, 4-hydroxy-cyclohexyl, or -CH₂-C≡CH.

2. The compound of claim 1, wherein one of R³, R⁴, and R⁵ is Vₚ-R', wherein, R' is an optionally substituted 5- or 6-membered fully unsaturated monocyclic ring having 0-3 heteroatoms independently selected from nitrogen, oxygen, or sulfur, or an optionally substituted 9- or 10-membered fully unsaturated bicyclic ring system having 0-5 heteroatoms independently selected from nitrogen, oxygen, or sulfur; or wherein one of R³, R⁴, and R⁵ is Vₚ-R', wherein R' is an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12-membered saturated, partially unsaturated, or fully unsaturated bicyclic ring system having 0-5 heteroatoms independently selected from nitrogen, oxygen, or sulfur.

3. The compound of any one of claims 1-2, wherein R⁴ is Vₚ-R', wherein R' is an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12-membered saturated, partially unsaturated, or fully unsaturated bicyclic ring system having 0-5 heteroatoms independently selected from nitrogen, oxygen, or sulfur; or wherein R⁴ is Vₚ-R', wherein R' is an optionally substituted C₁₋₆ aliphatic group or an optionally substituted 3-8-membered saturated, partially unsaturated, or fully unsaturated monocyclic ring having 0-3 heteroatoms independently selected from nitrogen, oxygen, or sulfur; or wherein R⁴ is Vₚ-R', and R' is C≡CH; or wherein R⁴ is Vₚ-R', and R' is an optionally substituted a 3-8-membered saturated, partially unsaturated, or fully unsaturated monocyclic ring having 0-3 heteroatoms independently selected from nitrogen, oxygen, or sulfur; or wherein R⁴ is Vₚ-R', wherein R' is an optionally substituted a 5-6-membered fully unsaturated monocyclic ring having 0-3 heteroatoms independently selected from nitrogen, oxygen, or sulfur; or wherein R⁴ is Vₚ-R', and R' is an optionally substituted a 6-membered fully unsaturated monocyclic ring having 0-3 nitrogen heteroatoms.

4. The compound of any one of claims 1-3, wherein p is 0; or wherein p is 1.

5. The compound of any one of claims 1-4, wherein R⁵ is halogen or Vₚ-R', wherein p is 0 and R' is hydrogen or C₁₋₆ aliphatic; or wherein R⁵ is halogen or Vₚ-R', wherein p is 0 and R' is hydrogen or C₁₋₃ alkyl.

6. The compound of any one of claims 1-5, wherein Ring A is:

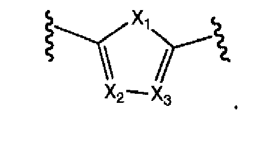

7. The compound claim 6, wherein ring A is: 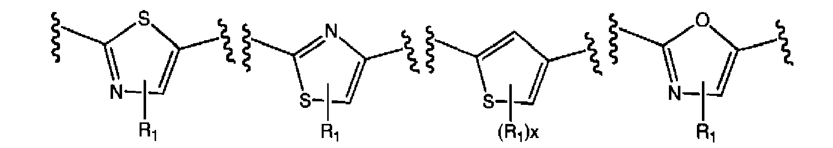 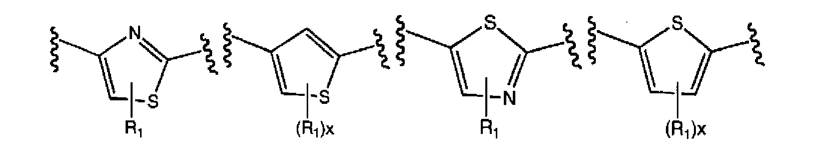 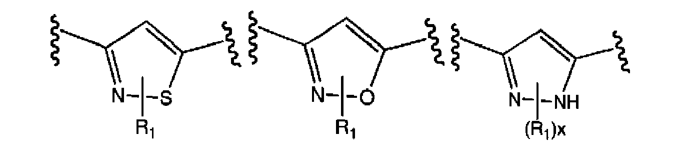 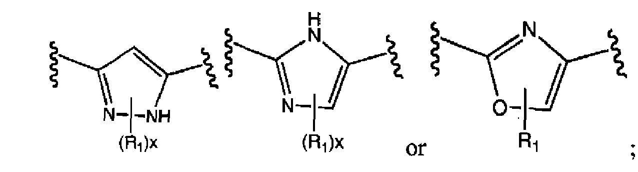 or wherein ring A is:

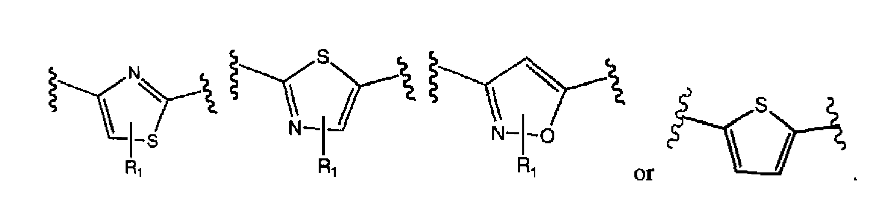

8. The compound of any one of claims 1-7, wherein R² is TₙR', wherein n is 1, and wherein T preferably is -NR-, -O-, -CO-, -CONR-, or -NRCO-.

9. The compound of claim 1, having a formula selected from 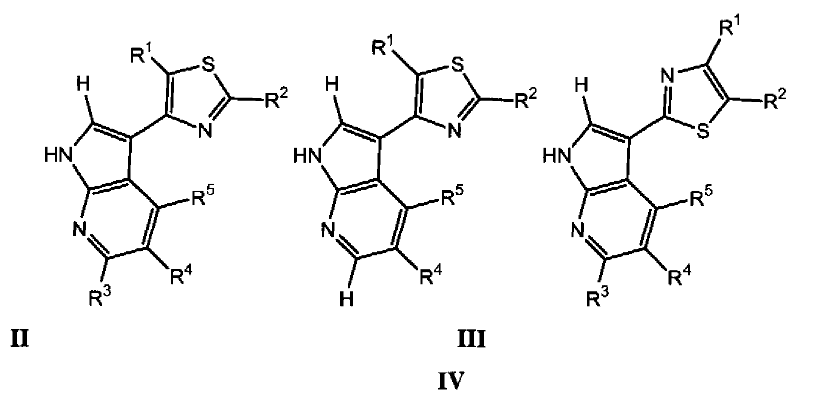 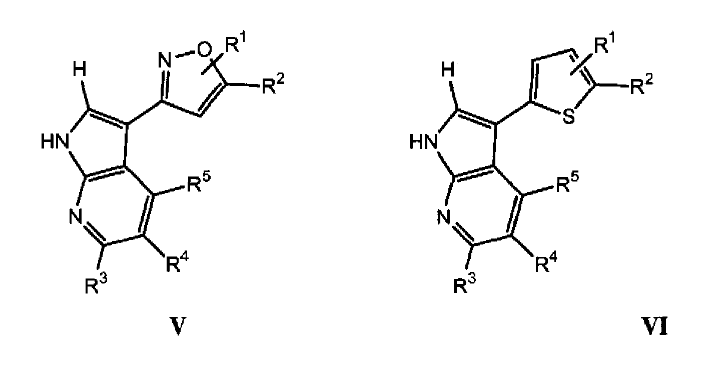 or a pharmaceutically acceptable salt thereof.

10. The compound of claim 9, wherein R² is TₙR', wherein n is 1; wherein T preferably is -NR-, -O-, -CO-, -CONR-, or -NRCO-.

11. The compound of claim 10, wherein T is -NR-; wherein R and R' preferably are both C₁₋₆aliphatic.

12. The compound of claim 9, wherein R² is TₙR', wherein n is 0; wherein R' preferably is an optionally substituted N-attached heterocyclyl selected from morpholinyl, piperidinyl, pyrrolidinyl, and piperazinyl.

13. The compound of any one of claims 9-12, wherein R⁴ and R⁵ are each independently Vₚ-R'; wherein R⁴ preferably is Vₚ-R', and R' is C≡CH.

14. The compound of any one of claims 9-12, wherein one of R³, R⁴, and R⁵ is Vₚ-R', wherein, R' is an optionally substituted 5- or 6-membered fully unsaturated (**i.e.**, **aromatic**) monocyclic ring having 0-3 heteroatoms independently selected from nitrogen, oxygen, or sulfur, or an optionally substituted 9- or 10-membered fully unsaturated bicyclic ring system having 0-5 heteroatoms independently selected from nitrogen, oxygen, or sulfur.

15. The compound of any one of claims 9-12, wherein one of R³, R⁴, and R⁵ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur.

16. The compound of claim 15, wherein R⁴ is Vₚ-R', wherein R' is independently an optionally substituted C₁₋₆ aliphatic group, an optionally substituted 3-8-membered saturated, partially unsaturated, or fully unsaturated monocyclic ring having 0-3 heteroatoms independently selected from nitrogen, oxygen, or sulfur, or an optionally substituted 8-12 membered saturated, partially unsaturated, or fully unsaturated bicyclic ring system having 0-5 heteroatoms independently selected from nitrogen, oxygen, or sulfur; and R³ and R⁵ is Vₚ-R', wherein p is 0 and R' is hydrogen; or wherein R⁴ is Vₚ-R', wherein R' is independently an optionally substituted C₁₋₆ aliphatic group or an optionally substituted 3-8-membered saturated, partially unsaturated, or fully unsaturated monocyclic ring having 0-3 heteroatoms independently selected from nitrogen, oxygen, or sulfur; or wherein R⁴ is Vₚ-R', and R' is an optionally substituted a 3-8-membered saturated, partially unsaturated, or fully unsaturated monocyclic ring having 0-3 heteroatoms independently selected from nitrogen, oxygen, or sulfur; or wherein R⁴ is Vₚ-R', wherein R' is independently an optionally substituted a 5-6-membered fully unsaturated monocyclic ring having 0-3 heteroatoms independently selected from nitrogen, oxygen, or sulfur; or wherein R⁴ is Vₚ-R', and R' is an optionally substituted a 6-membered fully unsaturated monocyclic ring having 0-3 nitrogen heteroatoms; or wherein R⁴ is Vₚ-R', and R' is an optionally substituted a 3-8-membered saturated monocyclic ring having 0-3 heteroatoms independently selected from nitrogen, oxygen, or sulfur; or wherein R⁴ is Vₚ-R', and R' is an optionally substituted a 6-membered saturated monocyclic ring having 0-2 heteroatoms independently selected from nitrogen, oxygen, or sulfur.

17. The compound of any one of claims 9-16, wherein p is 1; wherein V preferably is -NR-, -S-, or -O-.

18. The compound of any one of claims 9-17, wherein R⁵ is halogen or Vₚ-R', wherein p is 0 and R' is hydrogen or C₁₋₆ aliphatic; or wherein R⁵ is halogen or Vₚ-R', wherein p is 0 and R' is hydrogen or C₁₋₃ alkyl.

19. The compound of claim 1, selected from:

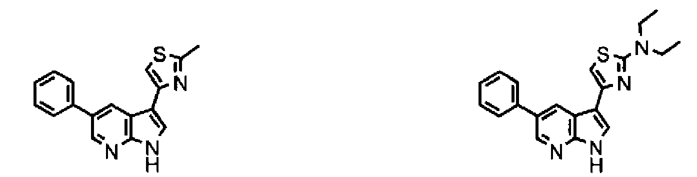

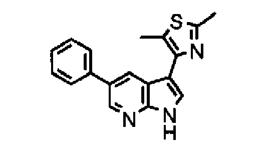

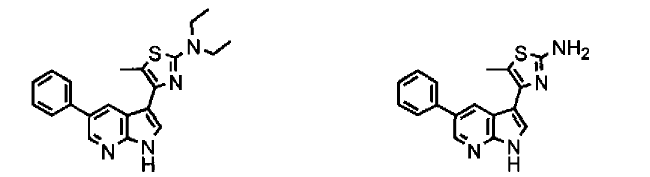

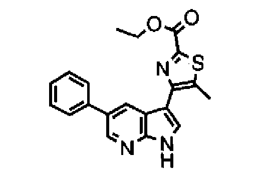

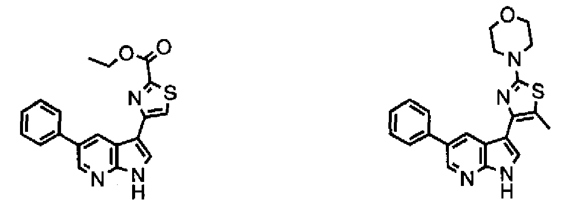

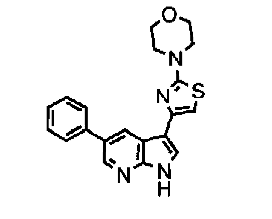

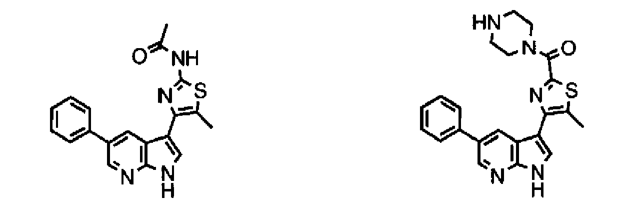

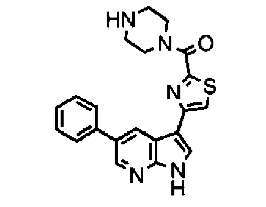

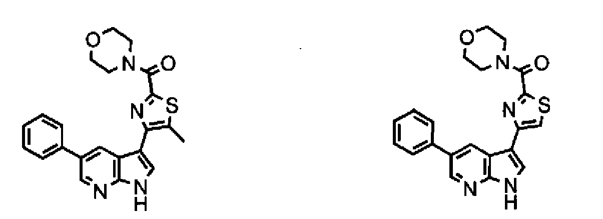

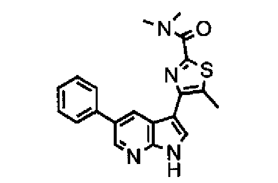

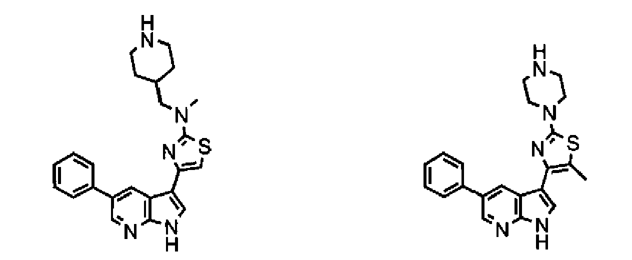

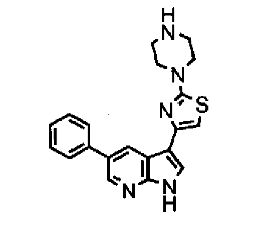

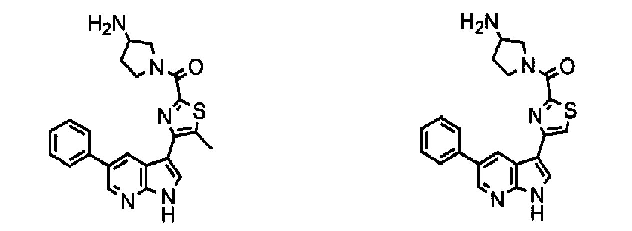

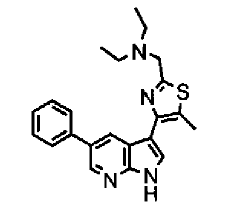

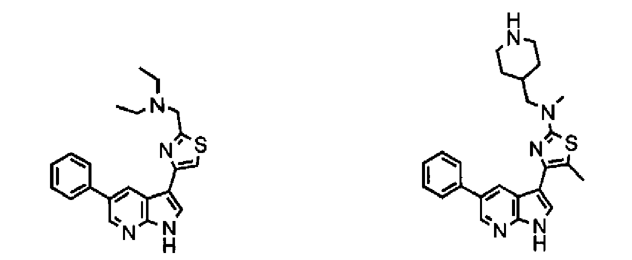

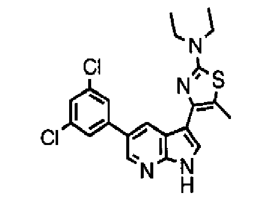

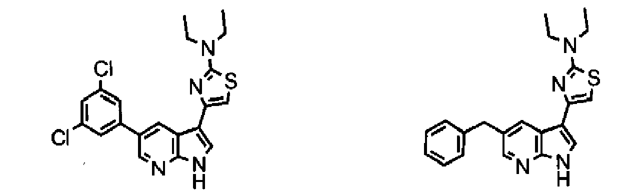

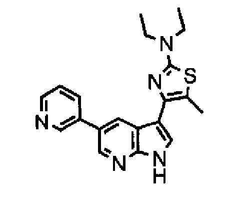

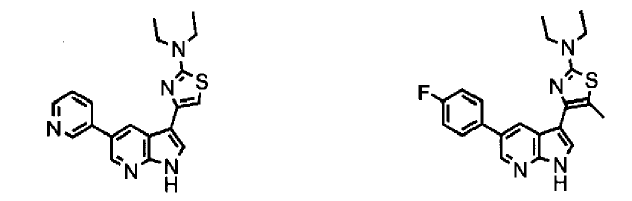

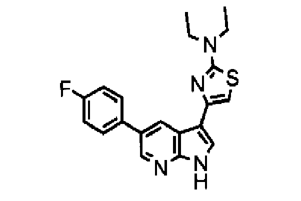

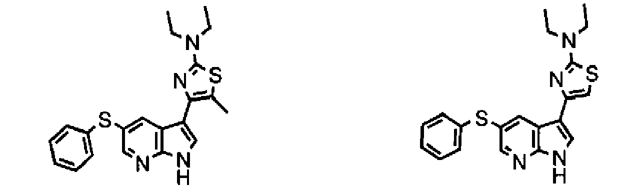

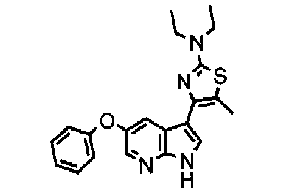

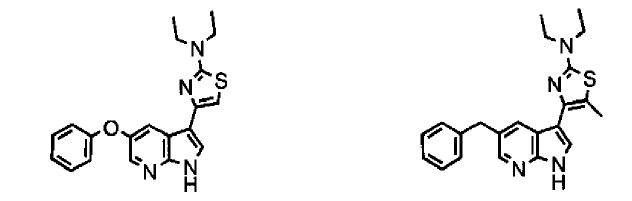

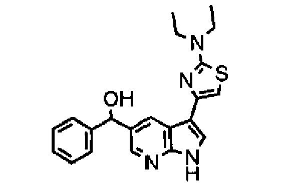

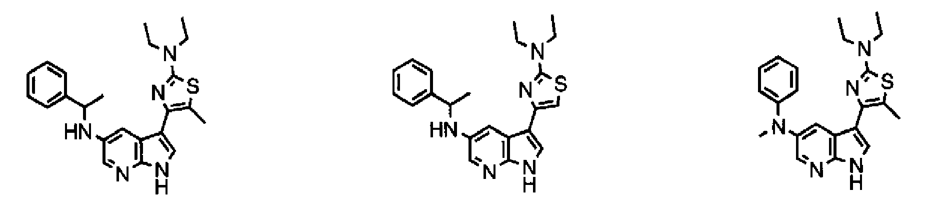

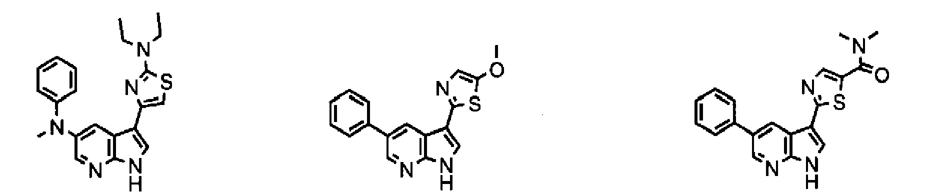

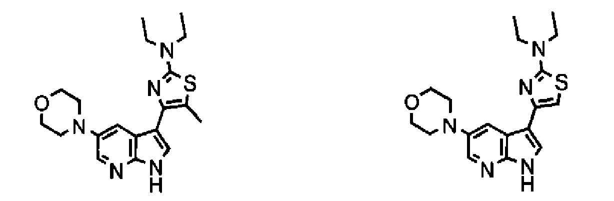

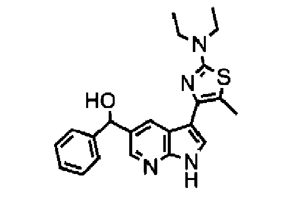

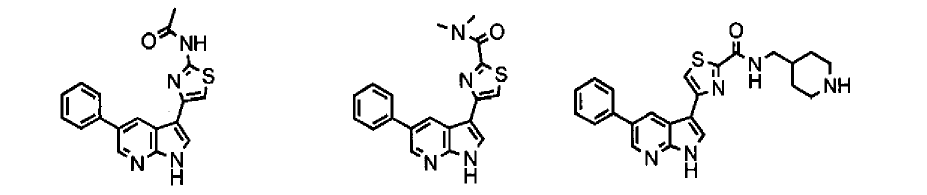

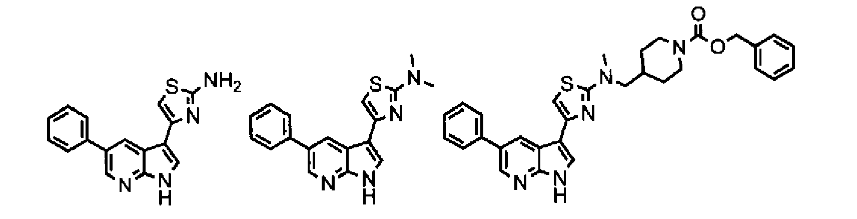

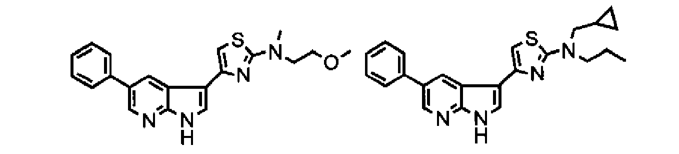

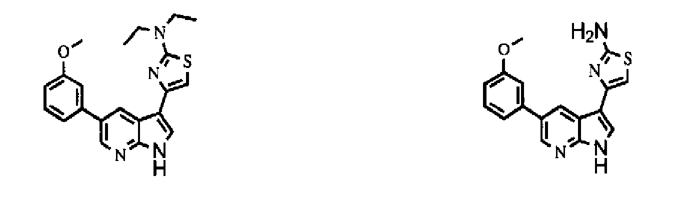

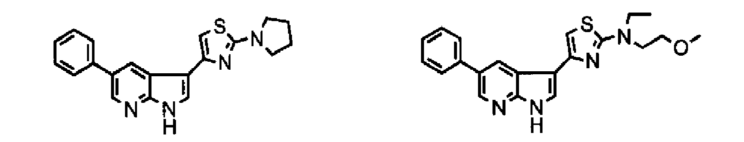

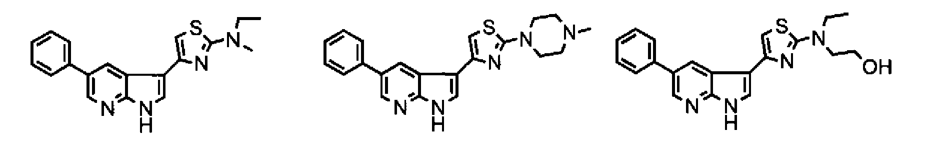

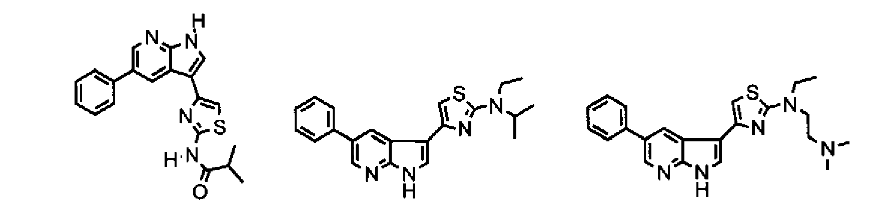

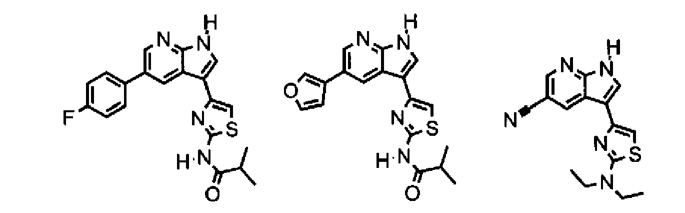

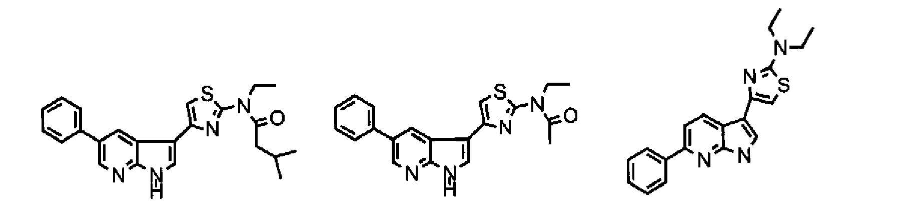

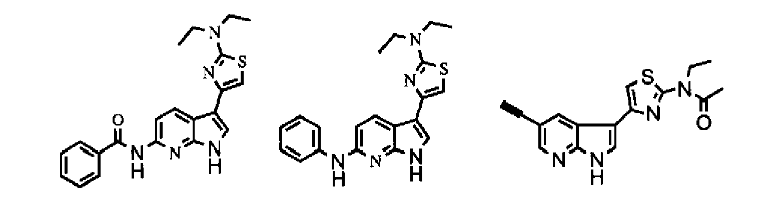

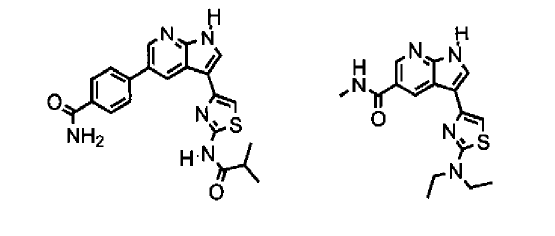

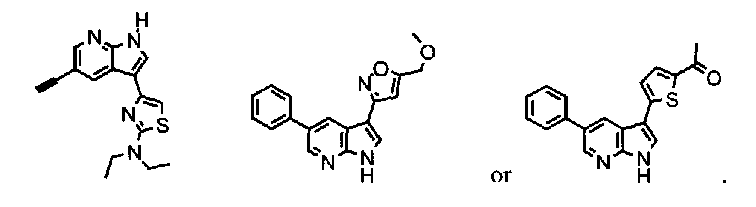

20. A composition comprising a compound of any one of claims 1-19 or a pharmaceutically acceptable salt thereof, and a pharmaceutically acceptable carrier or diluent.

21. A method of inhibiting Tec family (e.g., Tec, Btk, Itk/Emt/Tsk, Bmx, Txk/Rlk) kinase activity in:
a biological sample *in vitro;*
which method comprises contacting said biological sample with a compound according to any one of claims 1-19.

22. A composition comprising a compound according to any one of claims 1-19 for treating or lessening the severity of a disease of condition selected from an autoimmune, inflammatory, proliferative, or hyperproliferative disease; an immunologically-mediated disease; or asthma, acute rhinitis, allergic, atrophic rhinitis, chronic rhinitis, membranous rhinitis, seasonal rhinitis, sarcoidosis, farmer's lung, fibroid lung, idiopathic interstitial pneumonia, rheumatoid arthritis, seronegative spondyloarthropathis (including ankylosing spondylitis, psoriatic arthritis and Reiter's disease), Behcet's disease, Sjogren's syndrome, systemic sclerosis, psoriasis, systemic sclerosis, atopical dermatitis, contact dermatitis and other eczematous dermatitis, seborrhoetic dermatitis, Lichen planus, Pemphigus, bullous Pemphigus, epidermolysis bullosa, urticaria, angiodermas, vasculitides, erythemas, cutaneous eosinophilias, uveitis, Alopecia, areata vernal conjunctivitis, Coeliac disease, proctitis, eosinophilic gastro-enteritis, mastocytosis, pancreatitis, Crohn's disease, ulcerative colitis, food-related allergies, multiple sclerosis, artherosclerosis, acquired immunodeficiency syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia purpura, restenosis following angioplasty, tumours, artherosclerosis, systemic lupus erythematosus, allograft rejection including, without limitation, acute and chronic allograft rejection following for example transplantation of kidney, heart, liver, lung, bone marrow, skin and cornea; and chronic graft versus host disease.
